# EUROPEAN PATENT APPLICATION

(11) **EP 4 091 610 A1**
(43) Date of publication of application: **23.11.2022**
(21) Application number: 21174459.4
(22) Date of filing: 18.05.2021
(51) Int. Cl.: A61K 31/437, A61K 38/00, A61K 38/16

(54) **INHIBITORS OF DREAM COMPLEX ASSEMBLY AND/OR FUNCTION FOR USE IN REPAIRING DNA DAMAGE**

(71) Applicant: Schumacher, Björn, 50674 Köln (DE)
(72) Inventor: Schumacher, Björn, 50674 Köln (DE); Bujarrabal Dueso, Arturo, 50674 Köln (DE)
(74) Representative: Moré, Solveig Helga

(57) **Abstract**

The present invention relates to the pharmaceutically-induced enhancement of DNA damage repair in cells and potential therapeutic applications thereof. In particular, the present invention is directed to an inhibitor of DREAM complex assembly and/or function for use in repairing DNA damage in a cell of a subject. The inhibitor of DREAM complex assembly may be an inhibitory nucleic acid inhibiting the expression of a component of the DREAM complex or an antibody to a component of the DREAM complex. Further disclosed is an inhibitor of DREAM complex assembly and/or function for use in treating and/or preventing a condition associated with DNA damage in a cell in a subject, such as age-related diseases and symptoms of ageing, a progeroid syndrome, acute radiation syndrome, chronic radiation syndrome, Xeroderma pigmentosum, Nijmegen breakage syndrome, Fanconi anemia and ataxia. The invention further discloses a method for obtaining at least one cell with reduced DNA damage, wherein the method comprises steps of providing at least one cell, and treating said at least one cell with an inhibitor of DREAM complex assembly and/or function to repair DNA damage in said cell. Finally, the invention provides pharmaceutical compositions comprising the at least one cell obtainable by said method.

## Description

The present invention relates to the pharmaceutically-induced enhancement of DNA damage repair in cells and potential therapeutic applications thereof. In particular, the present invention is directed to an inhibitor of DREAM complex assembly and/or function for use in repairing DNA damage in a cell of a subject. The inhibitor of DREAM complex assembly may be an inhibitory nucleic acid inhibiting the expression of a component of the DREAM complex or an antibody to a component of the DREAM complex. Further disclosed is an inhibitor of DREAM complex assembly and/or function for use in treating and/or preventing a condition associated with DNA damage in a cell in a subject, such as age-related diseases and symptoms of ageing, a progeroid syndrome, acute radiation syndrome, chronic radiation syndrome, Xeroderma pigmentosum, Nijmegen breakage syndrome, Fanconi anemia and ataxia. The invention further discloses a method for obtaining at least one cell with reduced DNA damage, wherein the method comprises steps of providing at least one cell, and treating said at least one cell with an inhibitor of DREAM complex assembly and/or function to repair DNA damage in said cell. Finally, the invention provides pharmaceutical compositions comprising the at least one cell obtainable by said method.

DNA is constantly challenged by genotoxic stress both from exogenous and endogenous sources. It has been estimated that a mammalian cell experiences up to 10⁵ DNA lesions on a daily basis (Schumacher et al., 2021, Nature 592, 695-703). If left unrepaired, many of these DNA lesions will result in permanent mutations in the genome. While certain mutations in the DNA of germ cells may drive the evolutionary process, mutations in somatic cells are often associated with a decline of cellular function and predispose an organism to cancer and other genetic diseases.

Cellular DNA repair mechanisms are essential for removing a wide range of lesions that occur through genotoxic stress. In germ cells, DNA repair is highly accurate and efficient in order to maintain genome integrity, while in somatic cell types DNA repair mechanisms effectively maintain genome integrity early in life but inefficiently operate during later, mostly post-reproductive stages (Gorbunaova et al., 2007, Nucleic Acids Res. 35, 7466-7474). Consequently, somatic mutation rates are orders of magnitude higher than germline mutation rates (Milholland, B. et al., 2017, Nat. Commun. 8, 15183).

Since time-dependent accumulation of somatic DNA damage and its consequences progressively hamper cellular functionality, DNA damage is considered to represent the main driver for the development of the chronic ailments associated with ageing. Interventions that aim at alleviating the root cause of ageing-associated multimorbidity should therefore be targeted by restoring genome integrity by reducing DNA damage and augmenting DNA repair (Schumacher et al., 2021).

Reducing exogenous DNA damage for example through UV protection and avoidance of tobacco smoking has already proven to lower ageing-associated disease risks. Dietary interventions might be able to curb some endogenous DNA damage sources, but the majority of spontaneous lesions will inevitably occur. Augmenting DNA repair has remained a great challenge due to the intricate complexity and variety of repair machineries. To overcome this obstacle, efforts have been made to identify cellular key regulators capable of simultaneously modulating multiple different DNA repair pathways to provide possible new targets for therapeutic interventions. However, such master regulators of DNA repair affecting multiple DNA repair systems have thus far remained elusive (Schumacher et al., 2021).

Therefore, in light of the state of the art, there is a need for the identification of novel therapeutic targets for improving DNA repair, preferably, whose selective inhibition contributes to improved and prolonged DNA stability to effectively delay the process of ageing and fight the development of age-associated diseases.

The problem is solved by the present invention, in particular by the subject matter of the claims.

In a first aspect, the present invention provides an inhibitor of DREAM complex assembly and/or function for use in repairing DNA damage in a cell of a subject.

The dimerization partner, Retinoblastoma (RB)-like, E2F and multi-vulval class B (DREAM) complex is an evolutionarily highly conserved protein complex comprising the retinoblastoma-like 1 and 2 proteins p107 and p130 (also known as RBL1 and RBL2, respectively), the two E2F transcription factors E2F4 and E2F5, the E2F dimerization partners DP1, DP2 and DP3 as well as the multi-vulval class B (MuvB) core complex consisting of LIN9, LIN37, LIN52 and RBBP4 as its components (Sadasivam and DeCaprio, 2013, Nature Reviews Cancer, 13(8), 585-595).

The DREAM complex plays an essential role during entry into and maintenance of cellular quiescence, mainly by repressing the expression of hundreds of cell cycle-associated genes. Accordingly, loss of DREAM complex assembly and activity results in de-repression of many of its target genes and drives cell proliferation (Litovchick et al., 2007, Molecular Cell 26(4), 539-551). DREAM repressor complexes where also identified, inter alia, in the fruit fly *Drosophila melanogaster* (known as dREAM) and the nematode *Caenorhabditis elegans* (known as DRM) (Litovchick et al., 2007). In humans, in addition to their highly conserved repressor function in maintaining quiescence, the components of the DREAM complex can also associate with other proteins and instead function as transcriptional activator during the cell cycle (Sadasivam et al., 2012, Genes Dev. 26, 474-489); Osterloh et al., 2007, EMBO J. 26, 144-157; Mannefeld et al., 2009, Cancer Res. 69, 4073-4080).

Based on computational DNA motif enrichment analyses in C. *elegans* and Chip-Seq analysis in quiescent human cells, the present inventors uncovered that the DREAM complex also targets the promoters of a plethora of genes involved in the response to DNA damage, in particular a wide variety of genes required for effective DNA repair. While DREAM has in the past been linked to the resolution specifically of the rarely occurring DNA crosslinks and homology-directed double-stand break repair (Engeland 2018, Cell Death and Differentiation 25, 114-132, Chen et al., 2021, bioRxiv), the apparent extent of DREAM complex-dependent DNA repair regulation across distinct pathways was completely unexpected. The DREAM complex thus represents a primary candidate for the long sought-after master regulator of DNA repair.

DNA, i.e., deoxyribonucleic acid, is a biopolymer formed of monomeric building blocks called nucleotides. Each nucleotide consists of the 5-carbon sugar deoxyribose, a phosphate group and one of the four different nitrogenous bases cytosine (C), guanine (G), adenine (A) or thymine (T). DNA usually exists in a double-stranded state due to the hybridization of two single-stranded DNA molecules via complementary base pairing, i.e. the nitrogenous base adenine present in one DNA strand pairs and forms hydrogen bonds with thymine in the opposing strand, whereas cytosine pairs with guanine. As a result, DNA takes a characteristic double-helical shape in cells. In rare cases, DNA may however also exist in a single-stranded state, e.g., as part of an intermediate structure formed during DNA transcription called R-loop and during DNA replication. DNA encodes the genetic information required for the development, functioning, growth and reproduction of an organism. Less than 5% of the total DNA in a cell codes for polypeptides, whereas the rest is believed to perform mainly regulatory functions during protein biosynthesis or no function at all. Within the nucleus of a eukaryotic cell, DNA is wrapped around complexes formed of histone proteins to create a structure known as chromatin that is further sub-divided into separate units known as chromosomes.

In the context of the present invention, the term DNA mainly refers to nuclear DNA, i.e., the DNA that is to be repaired is preferably present in the nucleus of a eukaryotic cell. However, other eukaryotic cell organelles are known to contain their own DNA, including mitochondria or, in plants, chloroplasts. As the enzymes involved in the repair of mitochondrial or chloroplast DNA are the same as those required for nuclear DNA repair, in other embodiments, the DNA that is to be repaired is mitochondrial or chloroplast DNA. Preferably, the inhibitor of DREAM complex assembly and /or function promotes repair of all types of DNA molecules present in a cell, including nuclear, mitochondrial or chloroplast DNA. In some embodiments, the DNA may also be or include a DNA that has been introduced into the cell by artificial means, e.g., via direct injection or a viral vector. Accordingly, the DNA molecule that is repaired may be an exogenous and/or synthetic DNA.

In the context of the invention, the terms "DNA damage" or "DNA lesion" are used interchangeably and refer to abnormal alterations of the chemical structure of a DNA molecule or its building blocks. If left unrepaired, many types of DNA lesions may give rise to mutations in the DNA, i.e., permanent alterations in the nucleotide sequence of the genome.

To cope with constant genotoxic stress, cells have evolved a variety of highly specific DNA repair mechanisms capable of selectively recognizing and resolving particular types of DNA lesions. For instance, base-excision repair (BER) detects and repairs DNA lesions that usually do not distort the helix-structure of the DNA, such as bases that have been damaged due to oxidation (e.g., 8-oxoguanine or 5-hydroxycytosine), alkylation (e.g., 3-methyladenine/-guanine) or deamination (e.g., hypoxanthine or uracil) as well as entire base losses (abasic sites). BER relies on specific DNA glycosylases for damage detection and resolves the vast majority of DNA damage that occurs in a cell. Many of the downstream factors involved in BER are also required for repairing DNA single strand breaks (SSBs). In contrast, nucleotide excision repair (NER) is specialized on repairing bulky, helix-distorting adducts such as cyclobutane pyrimidine dimers (CPDs) and pyrimidine (6-4) pyrimidone photoproducts (6-4PPs) that frequently arise due to exposure to ultraviolet (UV-) light. NER is further differentiated into transcription-coupled (TC-) NER or global genome (GG-) NER, dependent on the mechanism of damage recognition. As the name implies, post-replicative DNA mismatch repair (MMR) mainly corrects base mispairings as well as small insertions or deletions that occur, e.g., during DNA replication or recombination. DNA double-strand breaks (DSBs) are often considered to represent the most severe type of DNA damage as they result in chromosome breakages that, if left unrepaired, may cause the death of a cell. On the other hand, misrepairing of DSBs can result in chromosomal translocations that promote the development of various types of cancer. The two major pathways specialized on DSB repair represent non-homologous end joining (NHEJ) and homologous recombination-directed repair (HRR). While NHEJ is the main DSB repair pathway, it is considered to be a rather error-prone repair mechanism as it simply re-ligates the two breakage ends, thereby often generating small deletions or insertions into the repaired DNA. By comparison, the error-free, yet more time-consuming HRR DSB repair relies on a homologous repair template to resolve DSBs. It therefore is restricted to particular phases of the cell cycle when a homologous DNA repair template is present, i.e., S and G2. The repair template may be a sister chromatid or a homologous chromosome. Finally, DNA crosslinks such as interstrand crosslinks (ICL) are recognized by the Fanconi anemia (FA-) pathway and are subsequently repaired by a concerted effort of factors involved in NER and HRR (Chatterjee and Walker, 2017, Environ Mol Mutagen 58(5), 235-263).

On the basis of their computational analyses on potential DREAM targets involved in DNA repair, the present inventors made the observation that inhibition of DREAM complex function resulted in the de-repression and thus upregulation of many DNA repair genes with essential roles in all of the above described pathways. Accordingly, a loss-of function mutation of the gene encoding the C. *elegans* DREAM complex component LIN-52 significantly elevated the repair of a variety of different DNA lesions, including the repair of CPDs, DNA DSBs, base alkylations and DNA crosslinks. The inventors thus confirmed a previously unexpected function of the DREAM complex as a key master regulator of DNA repair, affecting multiple, if not all major DNA repair systems. It is this unforeseen key function of DREAM in global DNA repair regulation that made its targeted inhibition worth considering for the first time.

Therefore, in a preferred embodiment of the invention, inhibition of DREAM complex assembly and/or function via the use of a suitable inhibitor augments the cell's capacity to repair a wide variety of DNA damages selected from the group comprising cyclobutane pyrimidine dimers (CPDs), pyrimidine (6-4) pyrimidone photoproducts (6-4PPs), DNA alkylations, base oxidations, base deaminations, interstrand crosslinks (ICLs), DNA mismatches, base losses, DNA single-strand breaks (SSBs) and DNA double-strand breaks (DSBs).

In the context of the invention, "augmenting", "improving", "enhancing" or "increasing" DNA repair in a cell can mean that existing DNA lesions are resolved at a faster rate upon inhibition of DREAM complex function, i.e., more DNA lesions in a cell are repaired in a defined period of time upon administration of the inhibitor of DREAM complex assembly and/or function as compared to a corresponding cell that has not been brought into contact with said inhibitor. Alternatively, it may also mean that a cell that under normal circumstances would not repair a DNA damage initiates DNA repair once being exposed to the inhibitor of DREAM complex assembly and/or function. Use of the inhibitor of DREAM complex assembly and/or function may also prolong the time during which DNA damages are repaired. Finally, "augmenting", "improving", "enhancing" or "increasing" DNA repair may also mean that a DNA damage is repaired more accurately, e.g., by reducing the risk of introducing secondary damages or mismatches into the DNA during the course of repair.

An inhibitor is herein understood as any compound, substance or composition capable of inhibiting a biological process or a chemical reaction. In the context of the invention, the inhibitor is capable of inhibiting DREAM complex assembly and/or function. The terms "inhibition of", "to inhibit", "inhibiting" or "inhibited" DREAM complex assembly mean that the formation of the DREAM complex via interactions of its individual or partially pre-assembled building components is delayed, reduced or, preferably, entirely prevented in the presence of the inhibitor. "Inhibition" may also mean that the DREAM complex is at least partially assembled, however, only to an extent that keeps it incapable of fulfilling at least its function in repression of DNA repair or, optionally, any of its functions. In the context of the invention, inhibition of DREAM complex assembly may also be understood to mean that the formation of an intermediate protein complex may take place that is able to perform some, most or even all of the functions of a fully assembled DREAM complex with exception to the repression of target genes involved in DNA repair. This embodiment is preferred. It is clear that complete prevention of DREAM complex assembly is inevitably associated with a corresponding abrogation of its function, whereas delayed or reduced assembly may still allow for delayed or reduced DREAM activity. Therefore, inhibition of DREAM complex assembly, as defined herein, corresponds with inhibition of its function.

However, in some embodiments, DREAM complex function may be inhibited without inhibiting DREAM complex assembly. For instance, the inhibitor according to the invention may bind to the fully assembled complex in a fashion that interferes or effectively abrogates the function of the DREAM complex, e.g., by blocking the complex's DNA binding domain. The inhibitor may also interact with another factor capable of binding to the fully assembled DREAM complex to indirectly inhibit DREAM complex function.

Of note, in the context of the invention, DREAM complex function is understood to refer only to the complex's ability to repress DNA repair genes, i.e., to prevent or limit the expression of target genes involved in or required for the above mentioned DNA repair pathways, e.g., by binding to DNA regions that control or regulate their transcription such as their promoters. In other words, in preferred embodiments, an inhibitor of DREAM complex function may only inhibit DREAM complex-mediated repression of DNA repair genes, while still allowing for some, most or all other functions of the complex, e.g., the suppression of cell cycle genes. In other embodiments, inhibition of DREAM complex function may however refer to inhibition of all or essentially all known and currently unknown DREAM functions by the inhibitor. Analogous to the above-described inhibition of DREAM complex assembly, "inhibition of", "to inhibit", "inhibiting" or "inhibited" DREAM function is intended to mean that the complex's function is either delayed, reduced or completely prevented.

The inhibitor of DREAM complex assembly and/or function according to the invention is selected from the group comprising an inhibitory nucleic acid, an antibody and a small molecule inhibitor.

In one embodiment, the inhibitor of DREAM complex assembly and/or function is an inhibitor of DREAM complex assembly, e.g., an inhibitory nucleic acid inhibiting the expression of a component of the DREAM complex, an antibody to a component of the DREAM complex or a small molecule inhibitor directed against a component of the DREAM complex.

Preferably, the inhibitor of DREAM complex assembly is an inhibitory nucleic acid inhibiting the expression of a component of the DREAM complex, wherein typically, the inhibitory nucleic acid is an inhibitory RNA.

Most inhibitory RNAs rely on a process known as RNA interference (RNAi) to post-transcriptionally inhibit the expression of a gene of interest. During this process, the inhibitory RNA guides a protein complex to a target mRNA molecule that has been transcribed from the gene of interest. Binding of said target mRNA is mediated via complementary base pairing between the inhibitory RNA and the target mRNA and is thus sequence-specific. Upon binding to the target mRNA, the protein complex associated with the inhibitory RNA initiates mRNA degradation or inhibits its translation into protein. The inhibitory RNA can be, e.g., a short hairpin RNA (shRNA), a microRNA (miRNA) or a small interfering RNA (siRNA). miRNAs are usually endogenously generated within a cell from long precursor transcripts that form hairpin structures and are processed through endonucleolytic cleavage. Small interfering RNAs (siRNAs) rely on a similar biogenesis pathway as miRNAs, but originate from double-stranded RNA molecules. Similar to miRNAs, they rely on RNAi to prevent target mRNAs from being translated into protein. Short hairpin RNAs (shRNA) are artificially produced molecules that are introduced exogenously into cells or organisms to decrease expression of a gene of interest through the RNAi machinery (gene knockdown). All of these inhibitory RNAs have in common that, when fully matured, they are single-stranded and comparably short, with a length of usually less than 30 nucleotides (nt).

A person skilled in the art is aware of how to choose or specifically design an inhibitory RNA capable of binding and repressing an mRNA that encodes a component of the DREAM complex by using standard laboratory techniques. SiRNA-mediated knockdown of DREAM complex components is described e.g. in Litovchick *et al.,* 2007. By interfering with the protein biosynthesis of at least one of its components, assembly of the DREAM complex can be effectively prevented.

In an alternative embodiment, the skilled person may choose to design an inhibitory nucleic acid that is not necessarily an RNA, e.g., an antisense oligonucleotide targeted against the mRNA of a DREAM complex component to prevent its expression and, accordingly, DREAM complex assembly. Antisense oligonucleotides are short synthetic nucleic acids made either from DNA or RNA that sequence-specifically bind to target mRNAs via complementary base pairing to block their translation into protein. As in the case of inhibitory RNAs for use in RNAi-mediated gene knockdown, a person skilled in the art is aware of standard laboratory techniques well known to the field to choose or design appropriate antisense oligonucleotides that specifically target the mRNA of a DREAM complex component of interest.

The inhibitory nucleic acid may be targeted to an mRNA encoding any component of the DREAM complex. In a preferred embodiment, the inhibitory nucleic acid is directed to a single type of mRNA encoding one DREAM complex component. However, in alternative embodiments, the expression of more than one DREAM complex components, e.g., two, three, four or more DREAM complex components may be inhibited by a combination of inhibitory nucleic acids directed to multiple mRNAs. In yet another embodiment, inhibitory nucleic acids may be directed to all components of the DREAM complex to prevent its assembly. Accordingly, the inhibitor of DREAM complex assembly may be a composition comprising a mixture of different inhibitory nucleic acids directed to either the same or to different components of the DREAM complex. However, given that at least in humans, the individual DREAM complex components may also fulfil additional cellular functions independent from those performed as members of the DREAM complex, it may be preferable to target particular components rather than others. In a preferred embodiment, the DREAM complex component whose expression is to be inhibited by the inhibitory nucleic acid is LIN52.

The inhibitory nucleic acid may also be a transcription factor decoy (TFD) oligodeoxynucleotide capable of specifically targeting and neutralizing the DREAM complex and/or any of its individual or pre-assembled components. TFD-oligodeoxynucleotides are short double-stranded DNA molecules that comprise the consensus DNA binding site of a specific transcription factor in the promoter region of its target genes (Hecker et al., 2017, Biochemical Pharmacology 144, 29-34). In consequence, the transcription factor binds to the decoy oligodeoxynucleotide rather than to the promoters of its target genes and thus is no longer able to affect their transcription rate. For instance, the inhibitory nucleic acid may be a TFD-oligodeoxynucleotide comprising a cell cycle-dependent element-cycle genes homology region (CDE-CHR) DREAM complex-binding motif or any of the previously reported DREAM binding motifs corresponding to SEQ ID NOs:42-43 (Litovchick *et al*., 2007). Such a TFD-oligodeoxynucleotide would be an inhibitor of DREAM complex function, as it does not prevent DREAM complex assembly but rather interferes with the binding of the DREAM complex to its target DNA repair genes. In an alternative embodiment, the inhibitory nucleic acid may be a TFD-oligodeoxynucleotide comprising a binding motif of one or more of the components that make up the DREAM complex. For instance, the TFD-oligodeoxynucleotide may comprise one or more E2F-binding motifs that can be recognized, e.g., by one or more of the DREAM components E2F4, E2F5, DP1, DP2 and DP3. Targeting individual DREAM components with TFD-oligodeoxynucleotides may prevent the function of the entire DREAM complex or may even prevent DREAM complex assembly. The TFD-oligodeoxynucleotide may comprise a single transcription factor binding site or a combination of different binding sites that are directed to the same or multiple different transcription factors, e.g., multiple DREAM complex components. The TFD-oligodeoxynucleotide may furthermore be modified to enhance its biostability, i.e., to delay the rate of its degradation by, e.g., 3'-exonucleases. For instance, the TFD-oligodeoxynucleotide may comprise nucleic acid analogs containing modifications in the nucleobase, the sugar ring or the phosphodiester backbone, such as phosphorothioates, peptide nucleic acids (PNA) or locked nucleic acids (LNA).

In another embodiment, the inhibitor of DREAM complex assembly may be an antibody to a component of the DREAM complex.

An antibody (also known as immunoglobulin) is a natural or synthetic, typically Y-shaped protein that can selectively bind a target antigen. The term "antibody" may include both polyclonal and monoclonal antibodies. In addition to complete immunoglobulin molecules comprising both variable and constant regions, an antibody in the context of the invention may also be a fragment of an antibody, e.g., a Fab fragment or a Fab₂ fragment, a human or humanized antibody, a bivalent antibody or a single chain antibody, as long as it selectively binds the target antigen. The antibody may be chosen or designed using standard laboratory techniques to specifically bind to a component of the DREAM complex in a fashion that prevents the component's incorporation into the DREAM complex or the function of the DREAM complex, as described above. As a consequence, a functional DREAM complex can no longer be assembled. Similar to the inhibitory nucleic acid, the antibody may be targeted to any DREAM complex component of interest. Furthermore, it is possible to not only target a single component, but also more, e.g., 2, 3, 4 or more components of the DREAM complex with a combination of different antibodies. In one embodiment, a collection of different antibodies may be used to target every component of the DREAM complex. Alternatively, multivalent antibodies targeting different components of the DREAM complex may be used. Accordingly, the inhibitor of DREAM complex assembly may also be a composition of different antibodies directed to the same or to different components of the DREAM complex. In one embodiment, the antibody targets LIN52.

The inhibitor of DREAM complex assembly and/or function may also be a small molecule inhibitor directed against a component of the DREAM complex. In the context of the invention, a small molecule inhibitor of DREAM complex assembly and/or function refers to a compound with a molecular weight of 900 Da or less that is capable of inhibiting, i.e., reducing, delaying or preventing the assembly and/or function of the DREAM complex. The small molecule inhibitor may, e.g., bind to at least one, e.g., one, two, three, four or all components of the DREAM complex in a manner to completely or at least partially inhibit DREAM complex assembly so that it is no longer capable of repressing the expression of its target DNA repair genes. In another embodiment, the small molecule inhibitor may bind to a component of the fully assembled DREAM complex and inhibit at least said function of the entire complex, e.g., by preventing DNA binding of the DREAM complex. The inhibitor of DREAM complex assembly and/or function may be a single type of small molecule inhibitor or a composition comprising multiple small molecule inhibitors with different targets in the DREAM complex.

In yet another embodiment, the inhibitor of DREAM complex assembly may be a composition comprising several types of different inhibitors. For instance, the inhibitor of DREAM complex assembly may be a composition comprising one or more different inhibitory nucleic acids and one or more different antibodies that are collectively directed to the same or to different DREAM complex components. In one embodiment, the inhibitor of DREAM complex assembly and/or function may be a composition comprising one or more different inhibitory nucleic acids, one or more different antibodies and one or more small molecule inhibitors that are collectively directed to the same or to different DREAM complex components.

In another embodiment, the inhibitor of DREAM complex assembly is an inhibitor of DYRK1A.

Dual-specificity tyrosine phosphorylation-regulated kinase 1A (DYRK1A) is a protein kinase required for assembly of the DREAM complex by phosphorylating the LIN52 protein at serine residue 28. Accordingly, pharmacological inhibition of DYRK1A kinase activity or mutation of said phosphorylation site of LIN52 completely disrupts DREAM complex assembly (Litovchick et al., 2011, Genes & Development 25, 801-813). The present inventors made the observation that by inhibiting DYRK1A activity, the majority of DREAM-repressed DNA repair genes becomes up-regulated in quiescent U2OS cells. As a consequence, inhibition of DYRK1A increases the resistance of these cells to DNA damage-inducing UV irradiation and the alkylating agent methyl methanesulfonate (MMS). Furthermore, DYRK1A inhibition augments repair of various types of DNA lesions. This finding was indeed unexpected, as it contradicts a previous publication reporting a functional role for DYRK1A in DNA damage repair (Guard et al., 2019, Scientific Reports 9:6539). According to this study, DYRK1A is required to recruit the DNA damage response protein 53BP1 to DNA lesions and thus to initiate repair of the damage.

The inhibitor of DREAM complex assembly according to the invention may thus be a DYRK1A inhibitor such as an antibody, an inhibitory nucleic acid, or a small molecule inhibitor.

In one embodiment, the inhibitor is an antibody that specifically binds to the DYRK1A kinase in a fashion to prevent it from phosphorylating Ser28 of LIN52. Accordingly, antibody-bound DYRK1A is no longer able to initiate DREAM assembly. The antibody may either selectively inhibit LIN52 phosphorylation by DYRK1A or completely disrupt the kinase activity of DYRK1A so that it can no longer phosphorylate any of its targets. A suitable antibody may be chosen or specifically designed using standard laboratory techniques.

In another embodiment, the inhibitor of DREAM complex assembly is an inhibitory nucleic acid that is directed to an mRNA required to produce DYRK1A. The inhibitory nucleic acid may be selected from the group comprising an inhibitory RNA such as a miRNA, an siRNA or an shRNA, or an antisense oligonucleotide. In any case, the inhibitory nucleic acid is to be chosen or designed using standard laboratory techniques to selectively prevent translation of the target mRNA into the polypeptide that ultimately becomes the DYRK1A kinase. As a consequence, DYRK1A levels decrease in a cell treated with the inhibitory nucleic acid with respect to its corresponding levels in an untreated cell.

In yet another embodiment, the inhibitor of DREAM complex assembly may be a small molecule inhibitor of DYRK1A. In the context of the invention, a small molecule inhibitor of DYRK1A refers to a compound with a molecular weight of 900 Da or less that is capable of inhibiting, i.e., reducing or preventing the kinase activity of DYRK1A. The small molecule inhibitor of DYRK1A is capable of binding to the DYRK1A kinase in a fashion so that it is no longer capable of phosphorylating Ser28 of LIN52. The small molecule inhibitor may either selectively inhibit LIN52 phosphorylation by DYRK1A or, alternatively, completely disrupt the kinase activity of DYRK1A so that it no longer is able to phosphorylate any of its targets.

Many pharmacological small molecule inhibitors of DYRK1A are known from the state of the art (e.g., lonescu et al, 2012, Mini-Reviews in Medicinal Chemistry 12, 1315-1329; Yoon et al., 2020, Int. J. Mol. Sci.; WO 2020/069418 A1). According to lonescu et al, 2012, most DYRK1A inhibitors are ATP-competitors that act by binding within the DYRK1A kinase domain. Accordingly, in a preferred embodiment, the small molecule inhibitor of DYRK1A according to the invention competes with ATP to bind to the kinase domain of DYRK1A. In alternative embodiments, the small molecule inhibitor may also bind to other regions of the DYRK1A protein to prevent the functionality of the ATP-binding site as an indirect ATP-inhibitor. In yet another possible embodiment, the small molecule inhibitor of DYRK1A interacts directly or indirectly with the activation loop of the DYRK1A catalytic domain to inhibit DYRK1A autophosphorylation and thus its kinase activity.

Preferably, the small molecule inhibitor of DYRK1A is either a nitrogenous heterocycle or a polyphenol compound as described in lonescu et al 2012. The nitrogenous heterocycle DYRK1A inhibitor may comprise a monocyclic, a bicyclic, a tricyclic or a tetracyclic core or scaffold selected from the group comprising a pyrazolidine-dione, a pyrrole-dione, an imidazolone, a pyridine, a thia-diazole, a purine, a benzothiazole, a tetrabromobenzimidazole (TBI), a quinolone, a quinazoline, a quinoline, a pyrimidinylindol, an azaindole, an 1*H*-Imidazo[4,5-c]pyridine-2-yl)-3-amino-1,2,5-oxadiazole, a pyrazolo[1,5-a]-1,3,5-triazine, a β-carboline, or a quiazolinone.

In a preferred embodiment, the small molecule inhibitor of DYRK1A is selected from the group comprising epigallocatechin-3-gallate (EGCG), harmine, (1Z)-1-(3-ethyl-5-hydroxy-2(3*H*)-benzothiazoylidene)-2-propanone (INDY), 3,5-di(polyhydroxyaryl)-7-azaindole (DANDY), (3-(4-fluorophenyl)-5-(3,4-dihydroxyphenyl)-1H-pyrrolo[2,3-b]pyridine (F-DANDY), folding intermediate-selective inhibitor of DYRK1A (FINDY), GNF4877, Roscovitine, Purvalanol A, 4,5,6,7-tetrabromo-1*H*-benzotriazole (TBB), CR8, quinazolinone, quinalizarin, Apigenin, Flavokavain A, Emodin, a meriolin, Ageladine A or Leucettine L41. In a particularly preferred embodiment, the small molecule inhibitor of DYRK1A is EGCG.

Preferably, inhibition of DREAM complex assembly and/or function occurs transiently and, optimally, in a reversible manner, i.e., DREAM inhibition is not permanent. In one embodiment, DREAM inhibition lasts only for a few hours, e.g. for at least 1 h to 48 h, for 2 h-36 h, for 3 h-24 h, for 4 h-12 h, for 5-6 h. The data shown herein demonstrate that a treatment with inhibitors of the invention for 24 hours already has a significant beneficial effect on repairing DNA damage. Alternatively, the DREAM inhibition lasts for at least a day to about a month, 2-14 days, 3-10 days, 4-7 days or about 5 days. Accordingly, the subject is to be treated with the inhibitor of DREAM complex assembly and/or function for at least 1 h to 48 h, for 2 h-36 h, for 3 h-24 h, for 4 h-12 h, for 5-6 h, or, alternatively, for at least a day to about a month, 2-14 days, 3-10 days, 4-7 days or about 5 days. Transient inhibition of DREAM complex assembly and/or function may be achieved by various means. For example, an inhibitory RNA is preferably naturally degraded within a given time period inside the cell by RNA-degrading enzymes normally expressed by the cell. A protein inhibitor may be associated with a protein domain capable of triggering degradation or destabilization of the entire inhibitor protein in the absence of a stabilizing ligand. Degradation and/or destabilization domains of such kind are well known in the art and can include, e.g., a rapidly degradable E. coli-derived dihydrofolate reductase (ecDHFR)-domain or destabilization domains derived, e.g., from DHFR, FRB or FKBP12 (Iwamoto et al., 2010, Chem Biol. 17(9), 981-988, Levy et al., 1999, Eur J Biochem. 259(1-2), 244-252; Stankunas et al., 2003, Mol Cell. 12(6), 1615-1624). Transient inhibition of DREAM assembly and/or function may further be achieved by limiting the supply of the inhibitor to the subject or by restricting the expression, the delivery and/or the release of the inhibitor into the target cell of interest by a specifically chosen vector. The inhibitor preferably is not an irreversible inhibitor, however, irreversible inhibitors may also be used, because of the constant turnover of proteins, e.g., components of the DREAM complex in the cell.

A transient inhibition of the DREAM complex helps to minimize risks of long-term deregulation of the DREAM complex, but preferably allows for an enhanced DNA repair for as long as needed. This may depend on the condition treated. Periods of inhibition of the DREAM complex can be repeated at need, e.g., every week, every month, every two months, every three months, every four months, every six months or every year.

The inhibitor of DREAM complex assembly and/or function according to the invention may be administered directly to the subject in need thereof, e.g., intravenously, intramuscularly, intraperitoneally, subcutaneously, transdermally or transmucosally. Administration may be by a single administration, e.g., an injection or infusion. A slow release formulation may also be chosen if administration over a longer time period is desired.

In a preferred embodiment, the inhibitor of DREAM complex assembly and/or function may be targeted to the cell via a suitable cell-specific vector. The type of cell-specific vector depends primarily on the nature of the inhibitor used to inhibit the DREAM complex. An inhibitory nucleic acid is preferably inserted into a viral vector, e.g., an adenoviral vector, an adeno-associated vector (AAV), or a retroviral, e.g., lentiviral vector. A person skilled in the art will be able to design suitable viral vectors to comprise a cargo DNA sequence encoding the inhibitory nucleic acid based on general knowledge and the prior art. For example, a stretch of cargo DNA, i.e., a transgene, encoding an inhibitory RNA may be operably linked to a suitable promoter and inserted into the viral vector of choice. The promoter used to express the inhibitory RNA may be any constitutively active promoter known in the art, e.g., a Rous sarcoma virus (RSV) promoter, a human cytomegalovirus (CMV) promoter, an HIV promoter or a eukaryotic promoter such as e.g., EF1a, PGK1, UBC, or human beta actin. Alternatively, the promoter may be an inducible promoter that only drives expression of the inhibitory RNA in the presence or absence of a certain stimulus. For example, the inducible promoter may be a Tet-regulated promoter, i.e., it may comprise a tetracycline response element (TRE) that can be bound by a tetracycline transactivator (tTA) protein in the presence of tetracycline or an analogue thereof, e.g. doxycycline. The promoter may also be a cell-specific promoter which only drives expression in a particular type of cell.

The assembled viral vector is preferably pseudotyped, e.g., with a viral envelope glycoprotein or a capsid capable of binding to a receptor expressed on the surface of the cell of interest. The term pseudotyping refers to the generation of viral vectors that carry foreign viral envelope glycoproteins on their surface or are encapsulated in a capsid of a different virus or viral serotype to specifically target only particular cell types of interest. The viral vector particles are usually assembled inside suitable packaging cells known in the art according to standard laboratory techniques before they are administered to the subject.

Inhibitory nucleic acids may also be delivered to the cell of interest using a vector that is a non-pathogenic, genetically modified bacterium or yeast comprising a prokaryotic or eukaryotic vector. The vector comprises a DNA molecule encoding the inhibitory nucleic acid against a DREAM component operably linked to a promoter that controls expression of said inhibitory nucleic acid, as described above. The non-pathogenic bacterium is preferably invasive, i.e., it is capable of entering a host cell and may be derived, e.g., from a non-pathogenic strain of *Escherichia coli, Listeria, Yersinia, Rickettsia, Shigella, Salmonella, Legionella, Chlamydia, Brucella, Neisseria, Burkolderia, Bordetella, Borrelia, Coxiella, Mycobacterium, Helicobacter, Staphylococcus, Streptococcus, Vibrio, Lactobacillus, Porphyromonas, Treponema,* or *Bifidobacteriae.* Preferably, the prokaryotic or eukaryotic vector is specifically designed for targeted delivery to a cell of interest, e.g., by expressing a ligand on its surface that can be recognized by a receptor on the target cell.

Alternatively, the inhibitory nucleic is not expressed on site from an expression vector at the cell of interest, but instead is expressed *in vitro* before being packaged into a suitable vector vehicle and directly delivered to the target cell. This way, the time of nucleic acid-mediated target gene inhibition can be effectively limited.

A person skilled in the art is aware of various types of vectors capable of selectively targeting, e.g., an inhibitory nucleic acid, an antibody or a small molecule inhibitor to a cell of interest. In one embodiment, the vector may e.g., comprise a lipid membrane that encapsulates the inhibitor. For example, the vector may be a liposomal or an exosomal carrier comprising the inhibitor of DREAM complex assembly and/or function as a cargo and, optionally, expressing a targeting moiety on its surface. It may also be a bacterial minicell. These types of carriers have the ability to entrap both hydrophilic therapeutic agents within their central aqueous core or lipophilic drugs within their bilayer compartment. The targeting moiety may be, e.g., a cell-specific antibody or ligand such as, e.g., a small peptide, polysaccharide or nucleic acid that can be bound by a receptor expressed on the surface of the cell of interest. Use of such a liposomal, exosomal or minicell vector has the benefit of allowing for fusion of the vector with the lipid membrane of the cell of interest e.g., during endocytosis to allow for the release of the inhibitor of DREAM complex assembly and/or function into the cytoplasm of the cell.

In another embodiment, the vector may be a polymeric micro- or nanoparticle. The micro- or nanoparticle may be formed of any physiologically acceptable polymeric material known in the art, e.g., it may comprise a biopolymer such as polysaccharide selected from the group comprising cellulose, alginate or chitosan. The vector may also be a nanocarrier comprising, e.g., a hyperbranched dendritic polyglycerol polymer. Such a nanocarrier is an amphiphilic unimolecular nanocarrier of dendritic structure, and it comprises a dendritic core and at least one shell.

In one embodiment, the vector releases the inhibitor of DREAM complex and/or function at once, after the vector has reached its targeted destination inside the subject. In another possible embodiment, the vector may be configured in a fashion to allow for a sustained release of the inhibitor in a desired period of time. A person skilled in the art will be able to choose a suitable vector from the art depending on the type of inhibitor used, the route of administration, the respective target cell to which the inhibitor is to be delivered and the condition to be treated. In addition, based on the known state of the art, the skilled person will be able to further modify the vector for optimized targeted delivery and release of the inhibitor.

In principal, the cell comprising DNA damage that is to be targeted by the inhibitor of DREAM complex assembly and/or function can be any type of cell of any origin or from any tissue. However, the risk of undesired effects associated with DREAM inhibition is lowest if the cell in which the DNA damage is to be repaired is a cell that does not divide, i.e., the cell is in a reversible or irreversible non-proliferative state.

When cells divide and proliferate, they progress through a series of events that are collectively summarized as the cell cycle. The cell cycle is divided into two major phases, mitotic (M) phase and interphase. During M phase, the cell undergoes nuclear division by mitosis, in which the chromosomes in the nucleus are first separated into two identical sets. The nucleus then divides into two daughter nuclei, each containing one of the sets of chromosomes. Following mitosis, the two nuclei, the cytoplasm, the cell organelles as well as the cell membrane are divided in a process called cytokinesis. In consequence, at the end of M-phase, the mother cell has been divided into two identical daughter cells.

By comparison, interphase represents the period during which a cell is prepared for undergoing cell division. Interphase consists of three distinct phases called G1, S and G2. During G1, the newly formed daughter cells boost their overall biosynthetic activity to support a phase of rapid cell growth. During the ensuing S phase, the DNA in the cell nucleus is replicated so that each chromosome exist in the form of two identical chromatids. Finally, G2 further prepares the daughter cells for another M-phase by additional cell growth and elevated protein biosynthesis (https://en.wikipedia.org/wiki/Cell_cycle).

Progression through the cell cycle is tightly regulated by the action of specialized cyclins and cyclin-dependent kinases. Transition from G1 to S-phase and G2 to M-phase is controlled by check-points. During these check-points, cells are monitored for DNA damage. Only if existing DNA lesions are repaired, the cell is allowed to pass through these checkpoints and to progress to the next phase of the cell cycle. This way, it is ensured that only undamaged "healthy" cells multiply.

Instead of transitioning from G1 to S-phase, the cell may alternatively enter a dormant phase of cellular inactivity known as G0. In fact, at any given time point, the vast majority of cells in the human body resides in G0. If entry into G0 is reversible, the cell is in a state known as cellular quiescence. Entry into quiescence and the maintenance of the quiescent state relies in part on the activity of DREAM. DREAM represses hundreds of cell cycle genes that normally drive cell cycle progression (Litovchick et al., 2007).

Since quiescent cells are in a dormant state characterized by significantly reduced protein biosynthesis and an overall decrease in biological activity, also their DNA repair capacities are greatly attenuated compared to that of cycling cells. In addition, quiescent cells no longer pass through DNA damage check points that ensure a concerted DNA damage response. In consequence, quiescent cells accumulate increasing amounts of DNA lesions overtime.

Accordingly, in a preferred embodiment, the cell that is to be treated with the inhibitor of DREAM complex assembly and/or function is a quiescent cell.

One particular group of cells known to reside in a quiescent state for prolonged times are tissue-specific stem cells. A stem cell is a cell that is undifferentiated or only partially differentiated. Tissue-specific stem cells, also known as somatic stem cells or adult stem cells, are self-renewing, multipotent cells found throughout the developed body. They are capable of differentiating into specialized tissue cells to replace dying cells and ensure constant tissue regeneration. In principle, tissue-specific stem cells are capable of rapid proliferation to ensure self-renewal. However, most of the times, tissue-specific stem cells are in a state of quiescence where they await external cues to initiate cell division and differentiation.

Different types of tissue-specific stem cells comprise, e.g., hematopoietic stem cells (HSCs) that are capable of differentiating into all blood cells and are primarily found in the bone marrow, intestinal stem cells that produce the cells of the large and small intestines and reside at the base of the crypts of Lieberkühn, mesenchymal stem cells (MSC) that can differentiate into different cell types including osteoblasts, chondroblasts, adipocytes, neuroectodermal cells, and hepatocytes and may be isolated from various tissues accordingly, endothelial stem cells required for replenishing endothelial cells, or neural stem cells that reside in the adult brain and are capable of forming new neurons. A study on HSCs could demonstrate that quiescent HSCs accumulate increasing levels of DNA damage as they age, which is only repaired once these cells are driven into the cell cycle (Beerman et al., 2014, Cell Stem Cell 15(1), 37-50). This finding suggests that tissue-specific stem cells cannot rely on particularly sophisticated geno-protective mechanisms during quiescence to ensure their long-term functional potential. Indeed, many adult stem cells are highly sensitive to endogenous and exogenous sources of DNA damage (Vitale et al., 2017, Molecular Cell 66, 306-319). Therefore, in accordance with the present invention, exposure to an inhibitor of DREAM complex assembly and/or function will result in a de-repression of DNA repair genes, cause a boost of DNA repair and consequently, greatly enhance genomic stability of quiescent stem cells during ageing.

It may be particularly advantageous, and the effects may be particularly helpful, if the quiescent cell that is to be treated with the inhibitor of DREAM complex assembly and/or function is a tissue-specific stem cell. The tissue-specific stem cell may be selected from the group comprising an HSC, an MSC, an intestinal stem cell, a mammary stem cell, an endothelial stem cell, a neural stem cell, an olfactory adult stem cell, a neural crest stem cell and a testicular stem cell.

In another embodiment, the quiescent cell is a mature hepatocyte. Hepatocytes make up the vast majority of cells in the liver. Although normally in a quiescent state in the liver, mature hepatocyte retain a high proliferative potential (Fausto, 2004, Hepatology 39(6), 1477-1487). Since the liver is responsible for the degradation and excretion of various toxins from blood, hepatocytes are subject to high genotoxic stress. Accordingly, inhibition of DREAM complex assembly and/or function may further assist in repairing DNA lesions in mature hepatocytes to ensure prolonged liver function.

In yet another embodiment, the cell that is to be treated with the inhibitor of DREAM complex assembly and/or function is a fully differentiated cell.

Wherein quiescent cells reside in a state of reversible G0, fully differentiated cells oftentimes irreversibly exit the cell cycle and are thus prone to accumulate increasing amounts of unrepaired DNA damage over time. In consequence, these cells are subject to continuous functional decline or are completely lost with increasing age of a subject.

For instance, unrepaired DNA damage in fully differentiated neuronal cells may be a source of neurodegeneration. Therefore, in a preferred embodiment, the fully differentiated cell that is to be exposed to the inhibitor of DREAM complex assembly and or function is a neuronal cell. In another embodiment, said fully-differentiated cell may be a fully-differentiated kidney cell. In the context of specific cell types, e.g., neurons or kidney cells, promotors specifically active in such cells can e.g. be used to specifically achieve expression of inhibitory RNA or a protein inhibitor in said cells. Suitable neuron-specific promoters may include, e.g., the synapsin 1 promoter, the tubulin α1 promoter, a neuron-specific enolase promoter, the dopaminergic neuron-specific tyrosine hydroxylase promoter and the Ca2+/calmodulin-dependent protein kinase II (CaM kinase II) promoter. Suitable promoters for kidney-specific expression of the inhibitor may include, e.g., the Ksp-cadherin promoter and the podocin promoter. Of course, alternatively or additionally, the inhibitor, or a vector comprising or encoding it, may also be specifically targeted to the fully differentiated cells.

Reference to a cell is understood to refer to at least one cell, e.g., substantially all or all cells of a specific cell type in a subject, substantially all or all cells of a specific cell type in a tissue of the subject, substantially all or all cells of a specific differentiation stage in a tissue of the subject, or substantially all or all cells in a tissue. It is also possible to repair DNA damage in all or substantially all cells of a subject in accordance with the invention, but more specific targeting is preferred.

As described in detail herein, an inhibitor of DREAM complex assembly and/or function can be used to repair DNA damage in a cell of a subject. Prolonged genetic instability due to accumulating unrepaired DNA lesions is considered to be a major driver of organismal ageing and represents the etiology of various diseases.

Therefore, in one embodiment, the present invention is further directed to an inhibitor of DREAM complex assembly and/or function for use in treating and/or preventing a condition associated with DNA damage in a cell in a subject, wherein the condition is selected from the group comprising age-related diseases and symptoms of ageing, a progeroid syndrome, acute radiation syndrome, chronic radiation syndrome, Xeroderma pigmentosum, Nijmegen breakage syndrome, Fanconi anemia and ataxia, wherein DNA damage in a cell of the subject is repaired.

In other words, the present invention also discloses a method of treating and/or preventing a condition associated with DNA damage in a subject, wherein the condition is selected from the group comprising age-related diseases and symptoms of ageing, a progeroid syndrome, acute radiation syndrome, chronic radiation syndrome, Xeroderma pigmentosum, Nijmegen breakage syndrome, Fanconi anemia and ataxia, wherein the method comprises a step of administering to said subject an inhibitor of DREAM complex assembly and/or function.

In the context of the invention, treating a condition is to be understood to comprise a curative medical therapy of a subject with the intent to cure, ameliorate or stabilize a condition. The term "condition" refers to a disease, a syndrome, a disorder or a particular physiological state of an organism that is either manifested by distinct symptoms or can be diagnosed by using established markers capable of recognizing said state. In some embodiments, treating a condition is intended to mean that the progression of the condition is to be slowed, stopped or, preferably, reversed to allow for a perceivable improvement of the subject's well-being and health. Preventing a condition refers to precluding, averting, obviating, forestalling, stopping, or hindering said condition from happening in the first place. Preventing a condition thus also explicitly includes the prophylactic treatment of a subject.

The subject of the herein disclosed invention can be a vertebrate, such as a mammal, a fish, a bird, a reptile, or an amphibian. Preferably, the subject of the herein disclosed methods is a mammal, e.g., a human, non-human primate, horse, pig, rabbit, dog, sheep, goat, cow, camel, cat, guinea pig, rat or mouse. In a particularly preferred embodiment, the subject is a human.

An age-related or ageing-associated disease refers to any disease that affects a subject primarily at an older age. In other words, the probability of contracting an age-related disease increases with the age of a subject. A symptom of ageing refers to any manifestation in a subject that is commonly associated with increasing age, e.g., frailty, sarcopenia, wrinkles in skin, hair loss, loss of hair pigmentation, or the slowing ability to heal e.g., fractures, atherosclerosis, rheumatoid arthritis, chronic kidney disease, type 2 diabetes, atrophy, multimorbidity, increased risk for cancer and neurodegenerative diseases but also less-apparent changes at the cellular level that underlie said visible manifestations, such as, e.g., entry of individual cells into cellular senescence as well as stem cell exhaustion.

It becomes increasingly clear that accumulation of unrepaired DNA damage in somatic cells is a major driver of the ageing process and thus also represents a main risk factor for contracting an age-related disease. Various cellular processes linked to ageing or the development and progression of age-related diseases may be impaired due to persistent DNA lesions, including telomere function, epigenetic modifications, proteostasis or mitochondrial function (Schumacher et al., 2021). Cellular senescence, which refers to an irreversible cell cycle arrest, is a cellular fate that results from excessive, unrepaired DNA damage and largely contributes to the ageing phenotype. Similarly, DNA damage promotes the exhaustion of tissue-specific stem cells both in number and functionality and reduces their multipotency, thus limiting tissue regeneration with increasing age.

In one embodiment, the ageing-associated disease and symptom of ageing is selected from the group comprising neurodegeneration, cancer and stem cell exhaustion.

Neurodegeneration refers to a progressive loss of structure or function of neurons as well as their death. There are many neurodegenerative diseases known and the development of most of these neurodegenerative diseases strongly correlate with the age of the subject.

Interestingly, many neurodegenerative diseases that are very different in their clinical manifestations have commonalities at the subcellular level, including the occurrence of cytotoxic misfolded proteins and aggregates in neuronal cells due to proteostatic stress. Multiple lines of evidence link DNA damage to the development of such proteostatic stress. Accordingly, DNA damage and altered expression and activity of DNA repair genes have been implicated in the pathogenesis of different types of dementias, including Alzheimer's disease or Parkinson's disease. Several DNA repair mechanisms, particularly mismatch repair, are also involved e.g., in the repeat expansion underlying Huntington disease and vice versa mutant huntingtin has been linked to defects in repairing transcription-associated DNA strand breaks (Schumacher et al., 2021).

Therefore, repairing DNA damage in neuronal cells by inhibition of DREAM complex assembly and/or function may prolong the functional lifespan of neuronal cells and protect a subject from neurodegeneration as he or she ages.

The inhibitor of DREAM complex assembly and/or function may alternatively be used for preventing the development of cancer. Cancer is defined as a group of diseases characterized by abnormal cell growth and the ability to invade (metastasize) into other tissues of the body. The major cause of cancer are mutations in genes that regulate cell cycle progression by e.g., activating cell cycle checkpoints or actively promoting cell proliferation. Similarly, chromosomal translocations that arise due to misrepaired DNA DSBs may alter the expression levels of genes and thus drive tumorigenesis. Accordingly, by reducing the overall levels of DNA damage in a cell in a subject using an inhibitor of DREAM complex assembly and/or function, it is possible to lower the overall risk of said subject to develop cancer. In principal, the cancer may be any cancer known in the art, including, e.g., breast cancer, lung cancer, colorectal cancer, liver cancer, or pancreatic cancer.

Of note, while Engeland (2018, Cell Death & Differentiation 25, 114-132) has associated inhibition of the DREAM complex with carcinogenesis, in the context of the present invention, a long-term inhibition of the DREAM complex that could promote unregulated cell division is preferably avoided, e.g., by targeting and/or regulating the inhibition of the DREAM complex as described herein. In addition, the subject that is to be treated with the inhibitor of DREAM complex assembly and/or function may be simultaneously administered an agent capable of preventing or delaying the re-entry of cells into the cell cycle upon DREAM inhibition, e.g. a cycline dependent kinase (CDK) inhibitor.

As mentioned above, stem cell exhaustion refers to a process where tissue-specific stem cells decline both in number and functionality and exhibit reduced multipotency. Stem cell exhaustion prevents proper tissue regeneration with increasing age.

In another embodiment, the condition to be treated and/or prevented according to the invention is a progeroid syndrome selected from the group comprising Werner syndrome (WS), Bloom syndrome (BS), Rothmund-Thomson syndrome (RTS), Cockayne syndrome (CS), trichothiodystrophy (TTD), combined xeroderma pigmentosum-Cockayne syndrome, Wiedemann-Rautenstrauch syndrome (WR) or Hutchinson-Gilford progeria syndrome (HGPS).

All of the progeroid syndromes listed above are rare genetic disorders that mimic symptoms of physiological ageing in children or young adults. They are thus also commonly referred to as premature ageing syndromes. Children diagnosed with these syndromes usually display a markedly reduced lifespan and an increased risk of developing cancer. WS, BS, RTS, CS, TTD and combined xeroderma pigmentosum-Cockayne syndrome all arise due to genetic mutations in genes that prevent normal DNA repair and thus result in abnormal accumulation of DNA damage at a very early age. For instance, WS, BS and RTS are caused by mutations in genes encoding RecQ helicases that function in DNA recombination, replication, repair and telomere maintenance. CS, TTD and xeroderma pigmentosum-Cockayne syndrome on the other hand develop due to defects in effective nucleotide excision repair. WS has been linked to mutations in the POLR3A gene encoding a subunit of RNA polymerase III. In addition, WS-patients suffer from increased levels of chromosomal breakage, indicative of defective DNA repair associated with this disease (Hou, 2009, Pediatr. Neonatol. 50(3): 102-109). Similarly, while HGPS is caused due to nuclear lamina dysfunction as a consequence of mutations in the LMNA gene encoding lamin A, the disease has previously linked to defects in DNA repair and increased genome instability (Gonzalo and Kreienkamp, 2015, Curr Opin Cell Biol. 34, 75-83).

Acute and chronic radiation syndromes may also be treated according to the invention. The terms acute and chronic radiation syndromes describe a variety of health effects that are caused due to high levels of severe DNA damage upon exposure to ionizing radiation (IR) such as X-rays or gamma-rays. Acute radiation syndrome (ARS) develops upon exposure to a total radiation dose of greater than 70 rad (0.7 Gy) that is delivered within very short time, i.e., within a few minutes. Classical immediate symptoms of ARS include nausea, vomiting, loss of appetite, and fever followed by characteristic skin changes in response to cutaneous radiation syndrome (CRS). Dependent on the dose of IR to which the subject has been exposed, death may occur within days or weeks. However, exposure to milder IR doses of less than 2 Gy may be treated with antibiotics, blood products, colony stimulating factors and stem cell transplants, and patients usually have good chances of survival. Application of an inhibitor of DREAM complex assembly and/or function boosts global DNA repair activities in the exposed subject, and therefore increases the chances of survival or reduces the risks of possible subsequent damages.

Chronic radiation syndrome (CRS) usually develops after months or years of chronic exposure to radiation. Symptoms of CRS include impaired senses of taste and smell, muscle and skin atrophy and an increased risk of developing cancer. Increasing DNA repair according to the invention may prevent or ameliorate symptoms of chronic and acute radiation syndrome. This is also one of the cases where prevention of mutations by increasing DNA repair is helpful, as, in some cases, exposure to radiation is foreseeable, e.g., in case of a subject who has to enter a radioactively contaminated area. In this case, the treatment is optimally begun before any symptoms of radiation syndrome occur, e.g., before or after the exposure.

Xeroderma pigmentosum is a disease caused by defective nucleotide excision repair and is characterized by a severe hypersensitivity to UV-light. Patients suffering from Xeroderma pigmentosum often develop sever sunburns after only a few minutes in the sun, changes in skin pigmentation and have a high risk of developing skin cancer and eye cataracts. Cockayne syndrome is caused by defective transcription-coupled repair and characterized by growth retardation and premature aging. Furthermore, nucleotide excision repair defects can cause trichothiodystrophy (TTD), Cerebro-oculo-facio-skeletal syndrome (COFS), and UV hypersensitivity syndrome (UVSS). Nijmegen breakage syndrome and Fanconi anemia are rare diseases characterized by ineffective HRR of DNA DSBs. Ataxia is characterized by a lack of voluntary coordination of muscle movements and can be triggered, inter alia, by radiation poisoning. A particular type of ataxia, ataxia-telangiectasia (AT), is associated with neurodegeneration as well as a predisposition to infection and is caused by mutations in the gene encoding the ATM DNA damage checkpoint kinase that is normally recruited primarily to sites of DNA DSB to initiate a DNA damage response. Accordingly, AT-patients also have an increased risk of developing cancers.

The inhibitor of DREAM complex assembly and or function used to treat and/or prevent any of the above mentioned conditions may be any of the herein described inhibitors. However, in one embodiment, the inhibitor of DREAM complex assembly and/or function for use in treating and/or preventing a condition associated with DNA damage is not a DYRK1A inhibitor, in particular when said condition is associated with a form of age-related neurodegeneration.

Since many of the above described syndromes and diseases are associated with defects in enzymes involved in particular DNA repair pathways, DREAM inhibition may compensate for the loss of these enzymes, e.g., by activating alternative repair pathways, for example by augmenting global-genome nucleotide excision repair to compensate for transcription-coupled repair defects or non-homologous enjoining to compensate for homologous recombination repair defects. However, in another embodiment, DREAM inhibition can also be performed concomitantly with a gene therapy aimed at producing the defect-free enzyme to further boost DNA repair.

In another embodiment, the present invention is directed to a method for obtaining at least one cell with reduced DNA damage, wherein the method comprises steps of:
a) providing at least one cell, optionally, from a sample from a subject, and
b) treating said at least one cell with an inhibitor of DREAM complex assembly and/or function to repair DNA damage in said cell.

The at least one cell may be any eukaryotic cell, i.e., of any origin, from any type of tissue and in any stage of the cell cycle. The cell may be provided, e.g., within a subject, wherein the subject is administered with an inhibitor of DREAM complex assembly and/or function, that, preferably, is targeted to the at least one cell inside the body of the subject as described herein.

In another embodiment, the at least one cell that is to be treated with the inhibitor of DREAM complex assembly and/or function has been isolated from a sample from said subject prior to the treatment, i.e., the cell is treated with the inhibitor of DREAM complex assembly and/or function outside of the subject's body. Accordingly, the method for obtaining the at least one cell with reduced DNA damage according to the invention is, preferably, an *in vitro* or ex *vivo* method. It is also possible to treat all or substantially all cells of a tissue or organ *in vitro* or ex *vivo,* e.g., in the context of an organ or tissue transplant. The organ or tissue may be an engineered tissue.

In the context of such an *in vitro* or ex *vivo* method, "treating" the at least one cell with the inhibitor of DREAM complex assembly and/or function according to step b) comprises contacting the cell with the inhibitor, e.g., in a suitable container, e.g., a test tube, that further contains a suitable, physiologically acceptable medium such as saline or water. The inhibitor may accordingly be taken up by the at least one cell by, e.g., endocytosis, transfection or transduction. In an alternative embodiment, "treating" may be understood to mean injecting the inhibitor of DREAM complex assembly and/or function directly into the at least one cell. As described above, the treatment of the at least one cell with the inhibitor of DREAM complex assembly and/or function may last for at least 1 h to 48 h, for 2 h-36 h, for 3 h-24 h, for 4 h-12 h, for 5-6 h, or, alternatively, for at least a day to about a month, 2-14 days, 3-10 days, 4-7 days or about 5 days

The at least one cell obtainable by the disclosed method is considered to exhibit "reduced DNA damage" when the cell has less DNA damage or is reasonably expected to have less DNA damage after its treatment with the inhibitor as compared to a corresponding non-treated cell. Based on general knowledge and the state of the art, a skilled person would be able to choose from numerous established methods to directly detect various types of DNA damage in a cell. Such methods include antibody staining for the presence of CPDs, 6-4PP or 8-oxoguanine. Other, less direct detection methods for DNA damage include COMET assay for assessing the levels of DNA single- and double-strand breaks, or high-performance liquid chromatography (HPLC) combined with mass spectrometric methodologies for detection of chemical nucleoside alterations or state-of the-art next generation DNA sequencing. The presence of DNA damage may also be assessed indirectly by antibody-mediated detection of indicators for active DNA repair. Such indicators include histone H2AX phosphorylation at serine139 (a state referred to as γH2AX), as well as foci formation of 53BP1, Rad51 and other repair or signaling proteins at DSB sites and sites of DNA damage-induced replication stress (Schumacher et al., 2021). In the context of the invention, the method of γH2AX detection is preferred for analyzing if a cell has reduced DNA damage.

Preferably, more than one cell, e.g., at least two cells, at least three cells, at least five cells, at least ten cells, at least 20 cells, at least 50 cell, at least 100 cells, at least 1000 cells, at least 1*10⁴ cells, at least 1*10⁵ cells, at least 1*10⁶ cells, at least 1*10⁷, at least 1*10⁸ or at least 1*10¹⁰ cells are treated with the DREAM inhibitor. In a particularly preferred embodiment, a population of cells comprising at least several hundred cells is treated in step b) of the herein claimed method. This would allow individual, randomly selected cells to be tested for DNA damage, and thus to deduce the average amount of DNA damage among all cells of the treated cell population. This way, the exact conditions of the treatment required for obtaining cells with reduced levels of DNA damage can be established and a suitable dose of the inhibitor required for increasing DNA repair in the at least one cell can be determined. The treatment effect will be assessed by expression changes of DNA repair genes. The effectiveness of the inhibitor treatments can furthermore be validated by apoptotic markers such as e.g., Annexin V, 7-ADD, and the levels of γH2AX following treatment with e.g., UV irradiation and other genotoxins.

The present invention is further directed to at least one cell obtainable by the above method. The at least one cell is characterized by reduced DNA damage compared to a corresponding cell that has not been obtained from the herein described method. In a preferred embodiment, said cell may be a stem cell, preferably a pluripotent stem cell, such as induced pluripotent stem cell.

The at least one cell may, for instance, be a pluripotent embryonic stem cell (ESC). ESCs will accumulate high levels of DNA damage when rapidly proliferating in cell culture. Despite the fact that ESCs appear to be able to mount very robust DNA damage responses and DNA repair, it may be possible to support genetic stability in cultivated ESC by exposing the cells *in vitro* to an inhibitor of DREAM complex assembly and/or function according to the above described method. As a result, the obtained ESCs exhibit decreased levels of DNA lesions as compared to corresponding cells that were not treated according to the method.

Induced pluripotent stem cells (iPSCs) are pluripotent cells that have been obtained by reprogramming of terminally differentiated cells via overexpression of defined groups of stemness-associated transcription factors such as OCT3/4, SOX2, KLF4, MYC, NANOG or LIN28A as well as stem cell-specific miRNAs. However, the clinical implementation of iPSC-based pharmaceutical compositions is currently limited as iPSCs intrinsically harbor high levels of DNA damage and an increased mutational load due to somatic mutations accumulated by the differentiated cells from which they were initially derived (Vitale et al., 2017, Molecular Cell 66, 306-319; Tapia and Scholer, 2016, Cell Stem Cell 19, 298-309). In addition, the reprogramming process itself is suspected to be mutagenic and may thus represent a source of de-novo DNA damage. It has further been suggested that prolonged culturing of iPSCs may introduce genetic alterations and that the *in vitro* pluripotent state of iPSCs promotes innate genetic instability (Liang and Zhang, 2013, Cell Stem Cell 13(2), 149-159). Therefore, in a particular preferred embodiment, an *in vitro* cultivated iPSC may be treated with the inhibitor of DREAM complex assembly and/or function according to the herein disclosed method to obtain an iPSC with reduced levels of DNA damage that is particularly safe for use in cell therapy. Alternatively, the at least one cell obtainable by the method of the invention may be a somatic cell with reduced DNA damage that is for reprogramming into an iPSC.

The at least one cell obtainable by the method may also be an adult or tissue-specific stem cell such as a neural stem cell, a mesenchymal stem cell or a hematopoietic stem cell. It may also be a fully differentiated or mature somatic cell such as a cardiomyocyte, a pancreatic islet cell or a chondrocyte. In another embodiment, the cell with reduced DNA damage obtainable by the herein described method is a lymphocyte such as a T-cell or a B-cell, e.g., a T-cell that has been genetically modified to recognize a cancer-specific antigen such as a chimeric antigen receptor (CAR-) T-cell generated for adoptive cell transfer. The at least one cell may also be a tissue or an organ, as described herein.

As a consequence of treating these cells with a DREAM inhibitor, they exhibit reduced levels of DNA lesions and are therefore particularly safe for use in cell therapy, i.e., a therapy during which viable cells are injected, grafted or implanted into a patient. Accordingly, the present invention further discloses a pharmaceutical composition comprising the at least one cell obtainable by the method described herein and, optionally, at least one pharmaceutically acceptable excipient. In a preferred embodiment, the pharmaceutical composition is for use in cell therapy. The cell may further be genetically engineered, e.g., to express a specific receptor such as a CAR, to reduce immunologic rejection and/or to correct a gene defect. For example, a pharmaceutical composition comprising at least one CAR-T cell that has been treated according to the method of the invention may be particularly safe for use for adoptive cell transfer to improve the immune functionalities of the recipient, i.e., the subject that is to be treated with the pharmaceutical composition. Alternatively, a pharmaceutical composition comprising an iPSC that has been obtained via the method of the invention may be administered to a subject so that the iPSC can promote tissue regeneration by differentiating into cells of interest. In another embodiment, a pharmaceutical composition comprising at least one cardiomyocyte obtainable by the method of the invention may be for use in, e.g., repairing heart function in a subject suffering from a heart disease. Transplantation of a pharmaceutical composition comprising at least one pancreatic islet cell obtainable by the method of the invention may be especially safe for use in treating a subject suffering from, e.g., diabetes.

Finally, a pharmaceutical composition comprising DREAM complex inhibitor-treated and thus genetically more stable neuronal stem cells may help to safely regenerate neuronal cells in the brain to, e.g., cure age-related neurodegenerative diseases such as Alzheimer's or Parkinson's disease. In principle, the pharmaceutical composition of the invention can therefore also be administered to a subject for treating a condition associated with DNA damage in said subject, wherein the condition is selected from the group comprising age-related diseases and symptoms of ageing, a progeroid syndrome, acute radiation syndrome, chronic radiation syndrome, Xeroderma pigmentosum, Nijmegen breakage syndrome, Fanconi anemia and ataxia, wherein DNA damage in a cell of a subject is repaired.

In summary, the present invention provides a novel pharmaceutical target that may be selectively inhibited to boost DNA repair function in somatic and quiescent cells. As described herein, the DREAM complex was found to be a key master regulator of all major DNA repair pathways. Although prolonged DREAM inhibition promotes cell proliferation and may thus favor tumour development and progression in the long-run, the unexpected novel key role of the DREAM complex in the modulation of DNA repair has the effect that at least a shortterm and/or targeted inhibition of this complex allows for efficient and thorough DNA damage resolution in cells. Accordingly, DREAM is considered to constitute a very attractive therapeutic target for fighting the root-cause of the ageing process and for treating many ageing- as well as DNA damage-associated diseases.

Throughout the invention, the term "about" is intended to be understood as "+/- 10%". If "about" relates to a range, it refers to both lower and upper limit of the range. "A" is intended to mean "one or more", if not explicitly mentioned otherwise.

All literature cited herein is herewith fully incorporated. The present invention is further illustrated, but not limited, by the following examples.

### Figure Legends

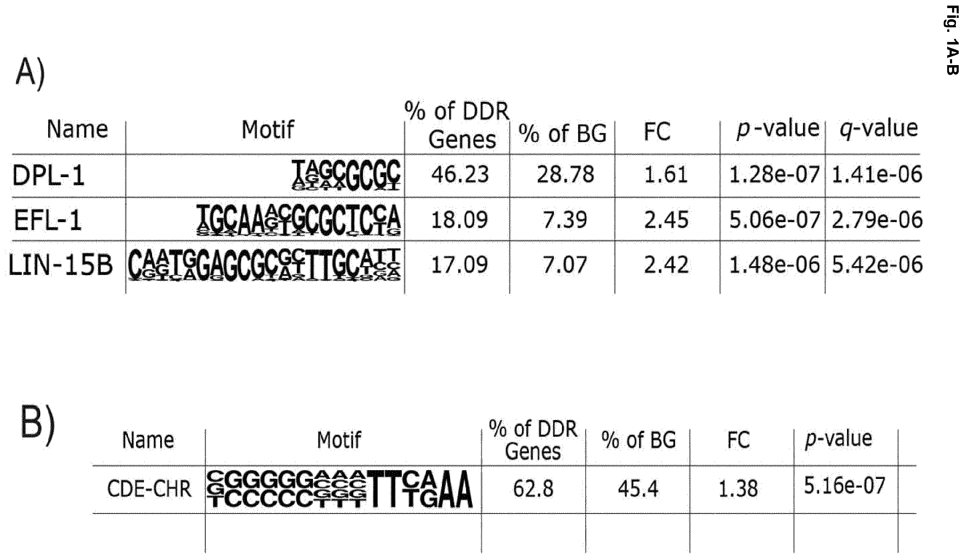

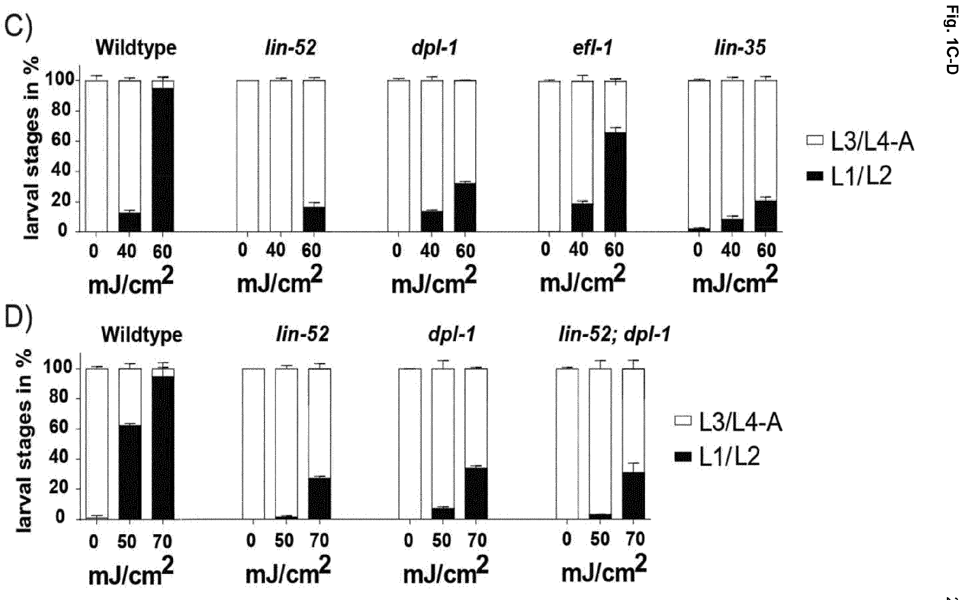

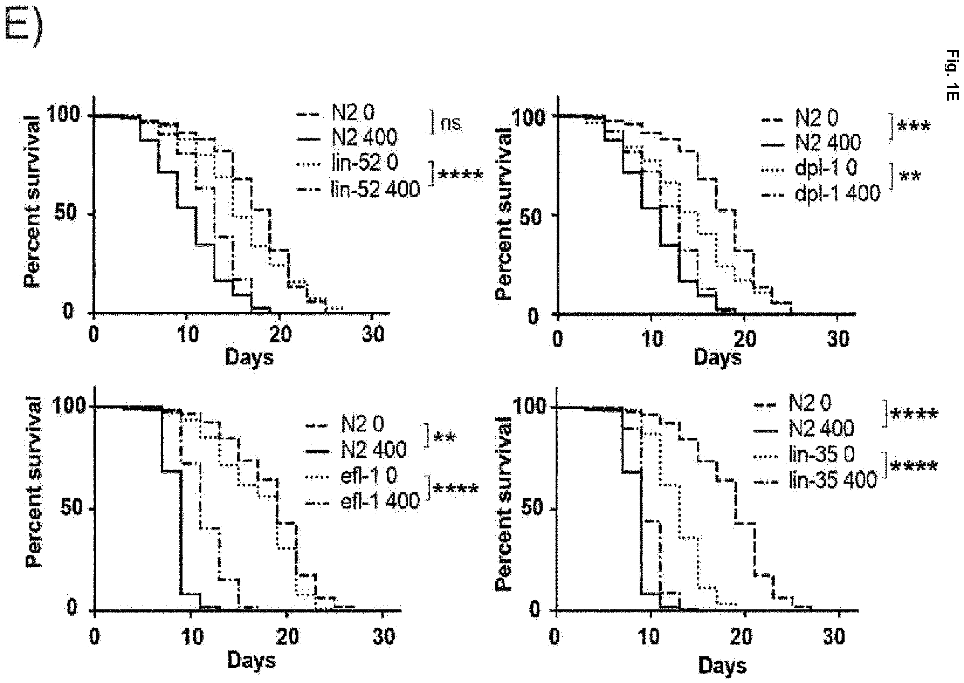

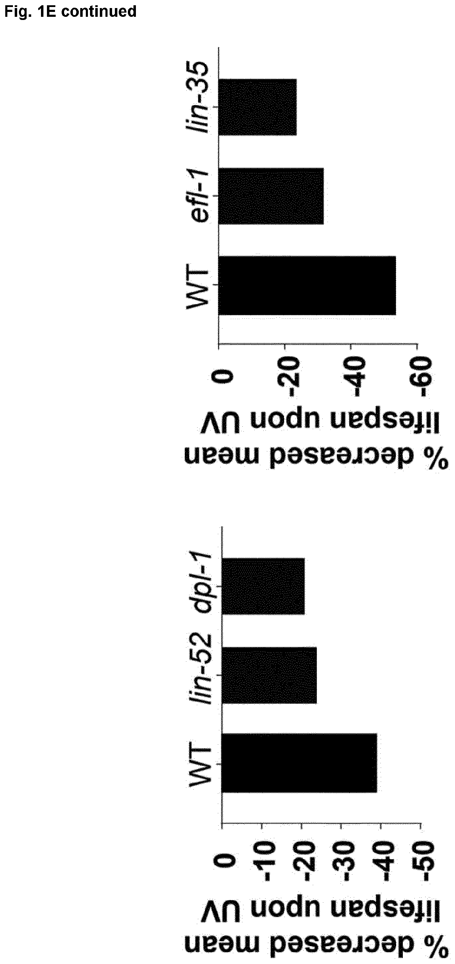

**Figure 1****. A-E Mutations of components of the DREAM complex confer resistance to UV-induced DNA damage during development and adulthood.**
   (A) Sequence logos of the motifs found upon analysis of the promoters of the 211 DDR genes using HOMER. A sequence logo is a graphical representation of the sequence conservation of nucleotides. A sequence logo is created from a collection of aligned sequences and depicts the consensus sequence and diversity of the sequences. The relative sizes of the letters indicate their frequency in the sequences. The total height of the letters depicts the information content of the position, in bits. HOMER called the DPL-1, EFL-1 and LIN-15B binding motifs as follows: DPL-1: TAGCGCGC, EFL-1: TGCAARYGCGCTCYA (SEQ ID NO: 38) and LIN15B: CARTGGAGCGCRYTTGCATT (SEQ ID NO: 39) (B) Result of the motif search of the CDE-CHR DREAM complex motif in the promoters of the DDR genes using HOMER. (C) L1 larvae of WT, *lin-52(n771), lin-35(n745), dpl-1(n2994)* and *efl-1(se1)* mutants were irradiated with UV-B or mock-treated and grown at 20 °C. Larval stages were determined 48 h post-irradiation. Representative graph from one out of at least three independent experiments. Mean of each larval stage out of three biological replicates per experiment; standard deviation (SD) between replicates is shown. Two-tailed *t*-test between the fraction of each larval stage of a mutant compared to WT in the same treatment condition showed p<0.01 upon UV treatment of each mutant to WT. n > 35 worms per replicate and condition. (**D**) L1 larvae of WT, *lin-52(n771), dpl-1(n2994)* mutants and the combined mutation of *lin-52(n771)* and *dpl-1(n2994)* were irradiated with UV-B or mock-treated and let to grow at 20 °C. Larval stages were determined after 48 h. Representative graph from one out of three independent experiments. Mean of each larval stage out of three biological replicates per experiment; standard deviation (SD) between replicates is shown. Two-tailed t-test between the fraction of each larval stage of a mutant compared to WT in the same treatment condition showed p<0.01 upon UV treatment of each mutant to WT but no difference between double mutant and single mutants. n > 37 worms per replicate and condition. (**E**) Day one adult worms were irradiated or mock treated with UV-B and incubated at 20 °C. Survival of the worms was analyzed every other day by discarding dead worms (immobile and unresponsive). log- rang (Mantel-Cox) test was performed to compare the lifespan of the DREAM mutants and WT in the same conditions. n > 128 individuals per condition and genotype. Bar-graphs show the % decreased mean lifespan of each strain irradiated with UV-B compared to the mock-treated worms of the same strain. * P < 0.05, ** P < 0.01, *** P < 0.001, **** P < 0.0001.

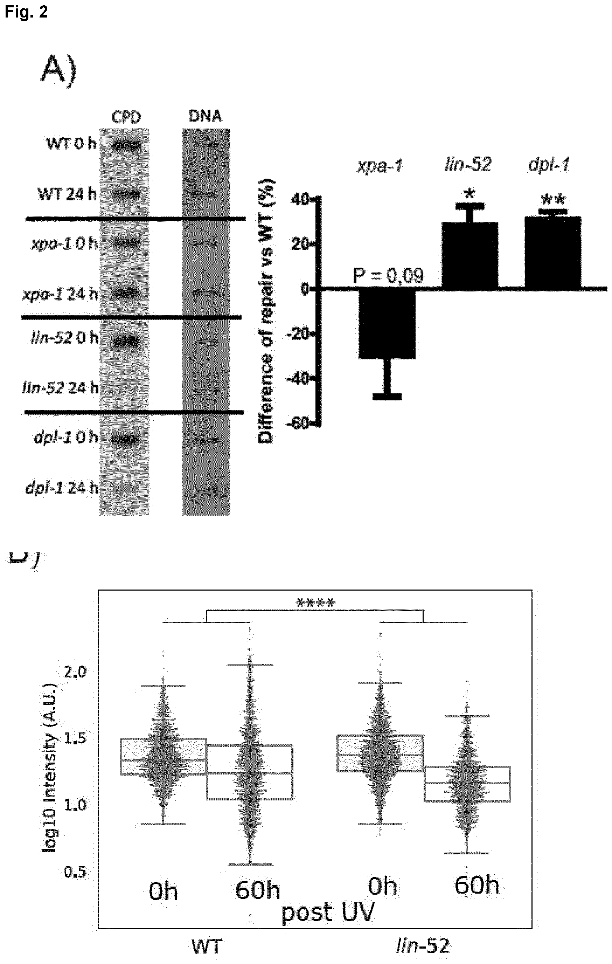

**Figure 2****. DREAM complex mutants show enhanced repair of UV-induced DNA lesions and alleviates the UV sensitivity of *csb-1* and *xpc-1* mutants.**
   (A) Representative slot blot out of three independent experiments labelled with antibodies against CPDs and SYBRTM Gold for DNA staining. DNA samples were collected from irradiated worms (WT, *xpa- 1(ok698), lin-52(n771)* and *dpl-1(n2994))* right after or 24 h after UV-B irradiation. Graphs represent the mean of the improved or decreased repair of the mutants tested compared to WT worms (mean with SD). Two-tailed *t*-test was used to compare the repair with WT worms. * P < 0.05, ** P < 0.01. (**B**) Quantification of CPDs nuclei signal intensity normalized with DAPI, in the heads of adult worms irradiated and collected immediately after or 60 h after irradiation. The number of nuclei quantified was n > 1300, from 5-7 heads per strain and condition. **** P < 0.0001.

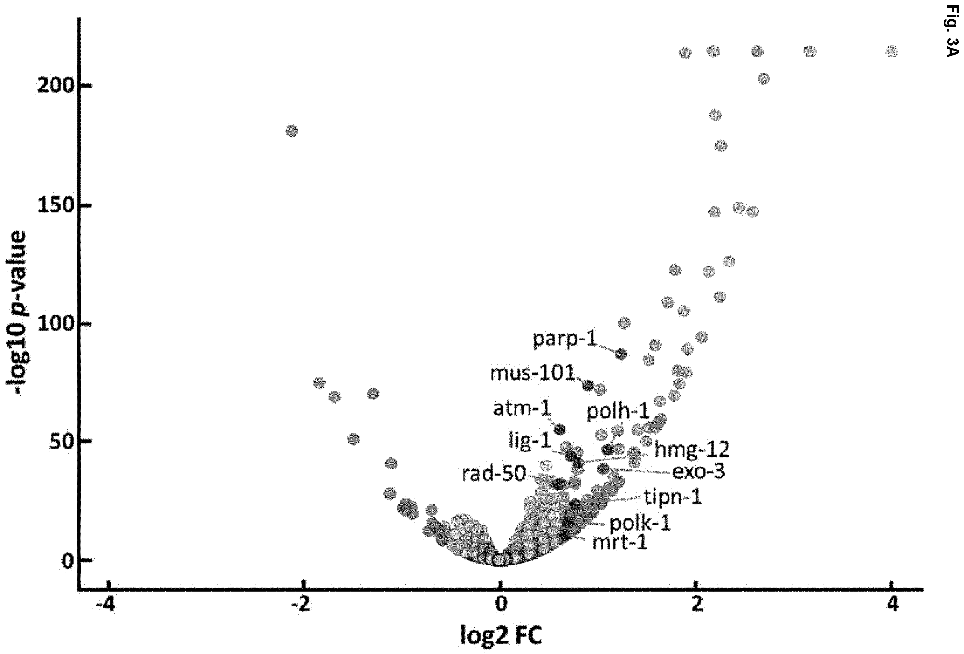

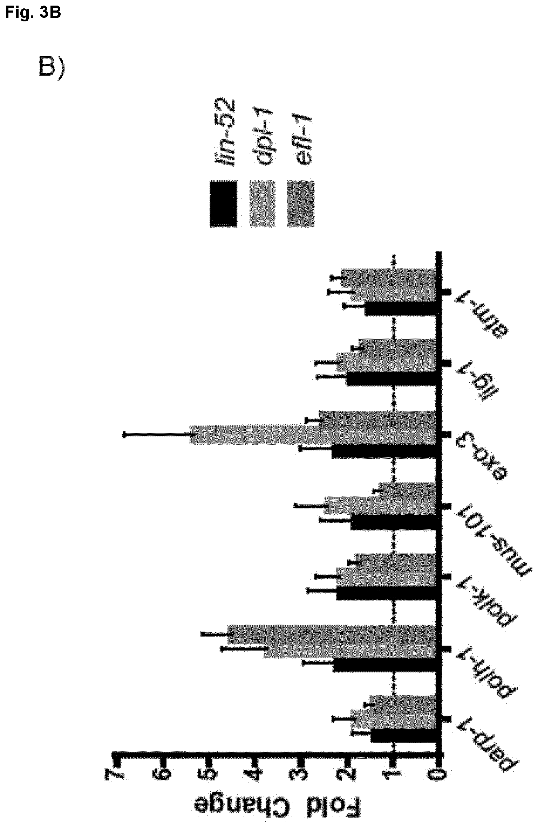

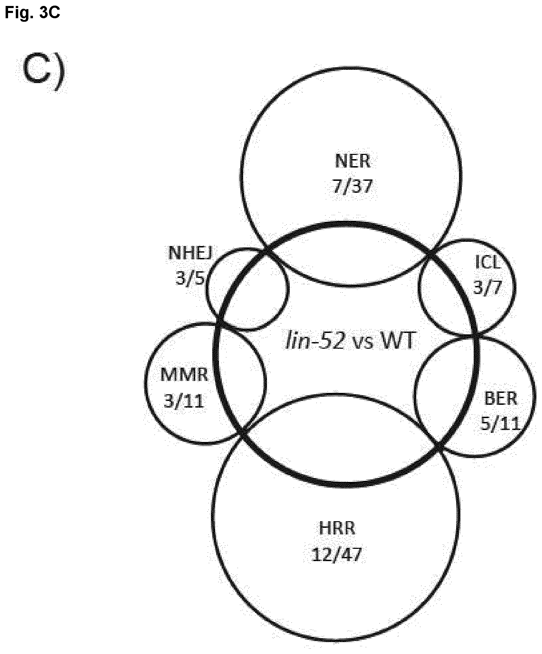

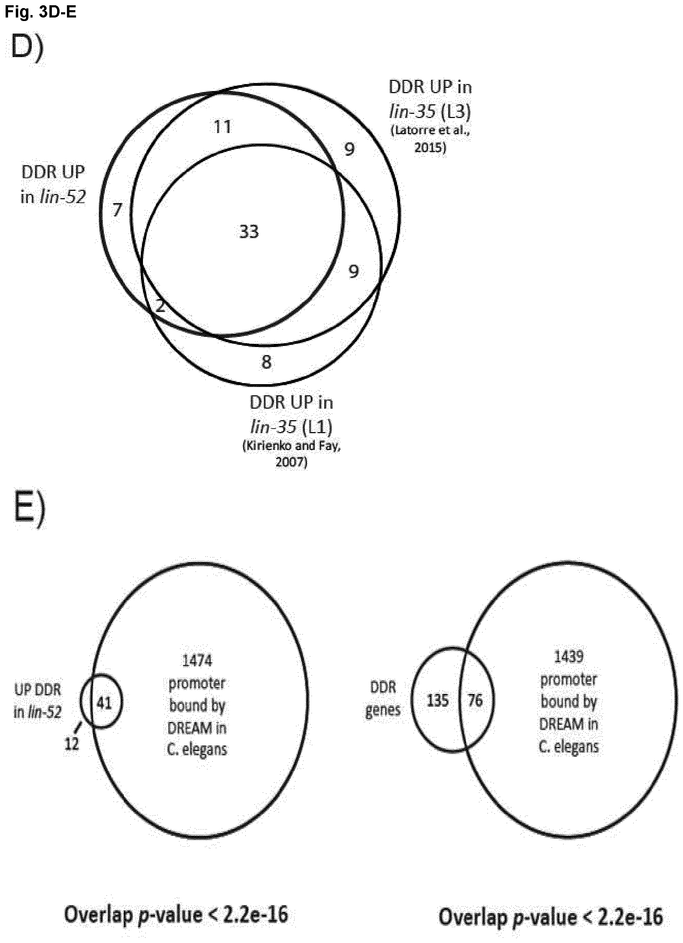

**Figure 3****. The DREAM complex directly represses multiple DNA damage response genes involved in the main DNA repair pathways.**
   (A) Volcano plot representing the RNAseq data of *lin-52* vs WT. In red and blue, all the genes with differential expression with a p-adj < 0.05 and FC < -1.5 and FC > 1.5 respectively. Highlighted in black are the genes belonging to the GO term "Cellular response to DNA damage stimulus" that were upregulated above 1.5 FC. (B) qPCR data showing that different mutations for the DREAM complex *(lin-52(n771), dpl-1(n2994)* and *efl-1(se1))* present an upregulation compared to WT of DDR genes that were previously found upregulated in the RNA-seq of *lin-52* vs WT. mRNA levels were normalized to the expression of the house keeping genes *eif3-c, Y45F10D.4* and *vha-6.* Graphs show the mean of three biological replicates with SD. (**C**) Area-proportional overlap between the genes upregulated in *lin-52(n771)* and the genes involved in the main DNA repair pathways (data from the GO consortium). The overlap between repair pathways is not represented; nucleotide excision repair (NER), interstrand crosslink repair (ICL), base excision repair (BER), homologous recombination repair (HRR), mismatch repair (MMR), non-homologous end joining (NHEJ). Pathway genes data from GO database released on 2019-10-08. (**D**) Area-proportional Venn diagram showing the overlap between the DDR genes found up-regulated in *lin-52* compared to WT worms and two published transcriptome datasets on *lin-35(n745).* (**E**) (Left) Venn diagram showing the overlap between the DDR genes found up-regulated in *lin-*52 compared to the genes that were found to be bound by DREAM complex in the promoter area (41 in promoter area, 43 in total). Re-analysis of reference 36. (Right) Overlap found between the genes bound by DREAM in the promoter area and all the DDR genes (Table S1) (76 in promoter area, 80 in total). Overlap p-value calculated by performing Fisher's Exact test.

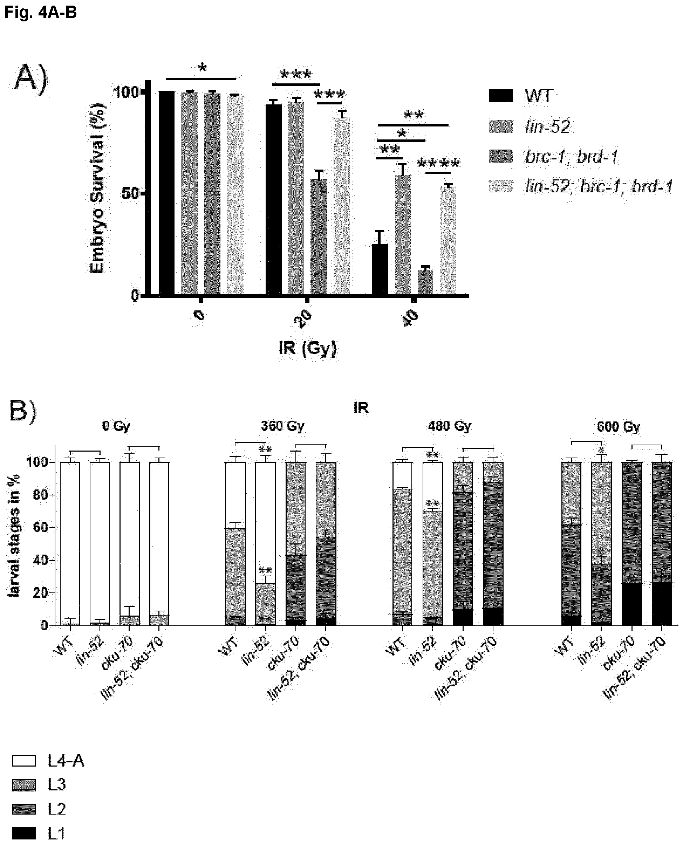

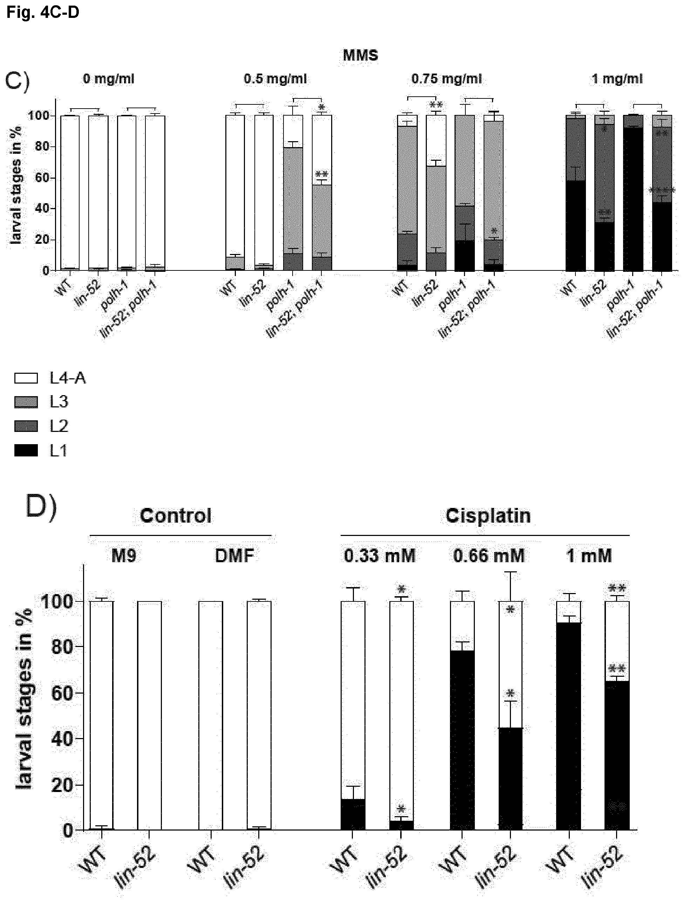

**Figure 4****. Mutations in the DREAM complex confer DNA damage resistance against multiple damage types.**
   (**A**) HRR in *lin-52(n771)* was evaluated by ionizing radiation (IR) treatment of early embryos of WT, *lin- 52(n771),* the HRR deficient worms *brc-1(tm1145); brd-1(dw1),* and *lin-52(n771); brc-1(tm1145); brd- 1(dw1).* The survival of the embryos was evaluated by the % of eggs hatched 24 h after irradiation. Representative graph of one out of three independent experiments, each with three biological replicates (mean with SD of the replicates). Two-tailed t-tests between the fraction of surviving embryos within the treatment are represented. n > 55 per replicate and condition. (**B**) NHEJ repair in *lin-52(n771)* was evaluated by irradiating L1 worms of WT, *lin-52(n771),* the NHEJ deficient worms *cku-70(tm1524)* and the double mutant *lin-52(n771); cku-70(tm1524),* and let to grow at 20 °C. Larval stages were determined after 48 h. Representative graph of one out of three independent experiments, each with three biological replicates (mean with SD of replicates). Two-tailed *t*-tests between the fraction of the larval stages of *lin-52* compared to WT, and *lin-52; cku-70* compared to *cku-70* (non-significant), are represented (showed statistics indicated with upper bracket). n > 31 per replicate and condition. (**C**) The response to alkylation damage in *lin-52(n771)* compared to WT was evaluated by treating L1 worms of WT, *lin-52(n771),* the alkylation damage sensitive animals *polh-1(lf31)* and double mutants *lin- 52(n771); polh-1(lf31)* to different doses of MMS, and let to grow after washing at 20 °C. Larval stages were determined after 48 h. Representative graph of one out of three independent experiments, each with three biological replicates (mean with SD of replicates). Two-tailed *t*-tests between the fraction of the larval stages of *lin-52* compared to WT, and *lin-52*; *polh-1* compared to *polh-1* are represented (showed statistics indicated with upper bracket). n > 20 per replicate and condition (average n = 55). (**D**) The response of *lin-52(n771)* to cisplatin compared to WT was evaluated by treating WT and *lin-52* L1 larvae with different concentrations of cisplatin (diluted in DMF), and let to grow after washing at 20 °C. Worms were also given the maximum dose of DMF that was given for the cisplatin treatments as control. Larval stages were determined after 48 h. Representative graph of one out of three independent experiments, each with three biological replicates (mean with SD of replicates). Two-tailed *t*-tests between the fraction of the larval stages of *lin-52* compared to WT are represented. n > 39 per replicate and condition. * P < 0.05, ** P < 0.01, *** P < 0.001, **** P < 0.0001.

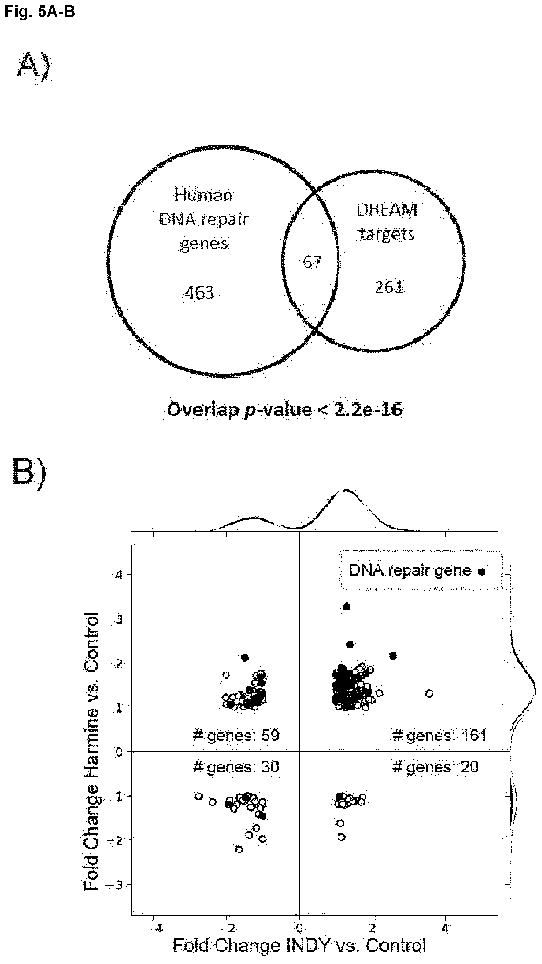

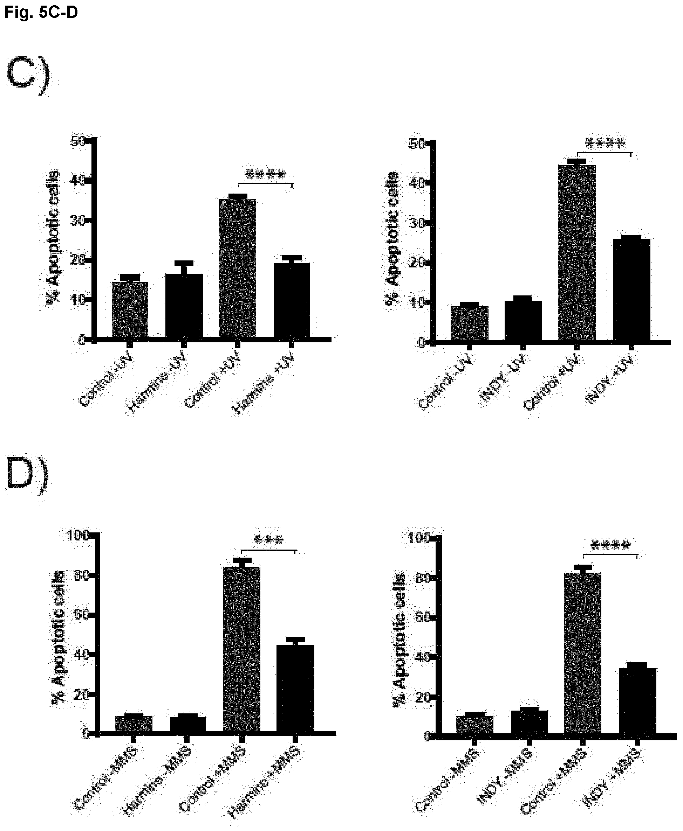

**Figure 5****. Inhibition of DREAM using DYRK1A inhibitors confer DNA damage resistance in human cells.**
   (**A**) Overlap between the 530 genes with GO term "DNA repair" in humans (Database released 2021-01- 01) and the 328 genes found to be bound by DREAM in humans upon re-analysis of Litovchick et al., 2007. Overlap p-value calculated using Fisher's exact test. (**B**) Derepression of DREAM targets by DYRK1A inhibitors. Plot of the 270 of the 328 DREAM target genes that present significant differences upon either harmine or INDY (FDR <0.01 for at least one of the two datasets) upon 24 h treatment. DNA repair genes are marked in orange, all remaining DREAM target genes are marked in blue. The distribution of either all 270 genes (grey), only the DNA repair gene subset (orange), or all DREAM target genes that are not included in the DNA repair gene annotation (blue) are shown on top and on the right side of the plot. The treatments show a strong overlap and derepression with 161 out 270 genes upregulated in both treatments (2.39 x over-enrichment, binomial *q*-value=9.56e-33). (C) Percentage of apoptotic U2OS cells (annexin V positive + annexin V positive / 7-AAD positive) upon harmine (left) or INDY (right) treatment and UV exposure (2 mJ·cm-2). Graphs show the mean with SD of a biological triplicate. Two-tailed *t*-tests between the populations under same irradiation conditions were performed. (**D**) Percentage of apoptotic U2OS cells (annexin V positive + annexin V positive / 7-AAD positive) upon harmine (left) or INDY (right) treatment and MMS treatment (2 mM for 2 hours). Graphs show the mean with SD of a biological triplicate. Two-tailed *t*-tests between the populations under same MMS conditions were performed. *** P < 0.001, **** P < 0.0001.

**Table 1: SEQ ID NOs.**

| **SEQ ID NO:** | Description | **SEQ ID NO:** | Description |
|---|---|---|---|
| **1** | Y45F10D.4 Forward primer | **22** | HPRT Reverse primer |
| **2** | Y45F10D.4 Reverse primer | **23** | GPDH Forward primer |
| **3** | eif-3.C Forward primer | **24** | GPDH Reverse primer |
| **4** | eif-3.C Reverse primer | **25** | CCNB1 Forward primer |
| **5** | vha-6 Forward primer | **26** | CCNB1 Reverse primer |
| **6** | vha-6 Reverse primer | **27** | FEN1 Forward primer |
| **7** | parp-1 Forward primer | **28** | FEN1 Reverse primer |
| **8** | parp-1 Reverse primer | **29** | PLK1 Forward primer |
| **9** | polh-1 Forward primer | **30** | PLK1 Reverse primer |
| **10** | polh-1 Reverse primer | **31** | POLQ Forward primer |
| **11** | polk-1 Forward primer | **32** | POLQ Reverse primer |
| **12** | polk-1 Reverse primer | **33** | RAD21 Forward primer |
| **13** | mus-101 Forward primer | **34** | RAD21 Reverse primer |
| **14** | mus-101 Reverse primer | **35** | RAD51 Forward primer |
| **15** | exo-3 Forward primer | **36** | RAD51 Reverse primer |
| **16** | exo-3 Reverse primer | **38** | EFL-1 binding motif |
| **17** | lig-1 Forward primer | **39** | LIN-15B binding motif |
| **18** | lig-1 Reverse primer | **40** | C. elegans CDE-CHR motif |
| **19** | atm-1 Forward primer | **42** | H. sapiens DREAM binding motif |
| **20** | atm-1 Reverse primer | **43** | H. sapiens DREAM binding motif |
| **21** | HPRT Forward primer | | |

### Examples

In the following examples, it is demonstrated in the nematode model organism *C. elegans* and in cultivated quiescent human cells how inhibition of DREAM complex assembly augments repair of various types of DNA lesions and confers resistance to genotoxic stress.

### Material and methods

### Promoter analysis - C. elegans

The set of 211 DDR genes was used as input for the findMotifs function of HOMER-4.11-2 (Heinz et al., 2010) with the parameters -len 8,10 -start -1000 -end 0. We first converted the Wormbase IDs to the sequence name with WormBase's SimpleMine (Harris et al., 2020) that were searched in the 'worm.description' file of HOMER to gain the corresponding RefSeq IDs. The p-values were calculated with the hypergeometric tests function in scipy(1.5.1). HOMER's seq2profile function (Heinz et al., 2010) was used to convert the previously reported CDE+CHR DREAM complex motif (Goetsch, Garrigues and Strome, 2017) with one mismatch and three random base pairs in between to a motif file usable by HOMER with the following parameter: seq2profile.pl BSSSSSNNNTTYRAA 1 (SEQ ID NO: 40). The constructed motif was searched with the findMo-tifs.pl -find function for the 211 DDR genes with the parameters -start -1000 -end 0. The back-ground enrichment of the motif was calculated for all 20174 protein-coding genes with a RefSeq ID included in the worm.description file of HOMER. The p-values were calculated with the hypergeometric tests function in scipy (1.5.1) (Virtanen et al., 2020).

### Promoter analysis - Human

Homer's seq2profile function was used to convert previously reported DREAM complex motifs (Litovchick *et al.*, 2007) with no allowed mismatch with the following parameter:
- seq2profile.pl TTTSSCGS 0
- seq2profile.pl WCGGAAGNB 0 (SEQ ID NO: 42)
- seq2profile.pl BNBVNTGACGY 0 (SEQ ID NO: 43)
- seq2profile.pl CWCGYG 0

The motifs were searched with the findMotifs.pl -find function with the parameters -start - 1000 -end 0 for the up-, respective down-regulated genes, after harmine, respective INDY treatment with an FDR-cutoff of 0.01. The background enrichment of the motif was calculated for all 20102 protein-coding genes included in the homer.description file of HOMER. The p-values were calculated with the hypergeometric test function in scipy(1.5.1) (Virtanen *et al*., 2020) and Python's statsmodels(0.11.1) (Seabold and Perktold, 2010) was used to calculate the Benjamini-Hochberg FDR.

### UV-irradiation for somatic development

The effects of UV-B in worm development were analyzed as previously described (Rieckher *et al*., 2017). Upon standard alkaline hypochlorite solution treatment to adult worms, embryos were left in a rotating mixer at room temperature over-night in M9 buffer. Hatched L1 worms were filtered using an 11 µm hydrophilic filter (Millipore, NY1104700), counted, and plated in NGM plates. Worms were mock-treated or irradiated with a 310 nm PL-L 36W/UV-B UV6 bulb. OP50 E. *coli* was added to the plates and worms were incubated at 20 °C for 48 h.

### IR sensitivity assay dependent on NHEJ repair

As previously described (Clejan, Boerckel and Ahmed, 2006; Johnson, Lemmens and Tijsterman, 2013), L1 worms repair DSBs mainly via NHEJ repair. Synchronized L1 worms were irradiated with different doses of IR using an IR-inducing cesium-137 source, and left 48 h at 20 °C to allow development.

### IR sensitivity assay dependent on HR repair

Early embryos highly rely on HRR to repair DSBs (Clejan, Boerckel and Ahmed, 2006; Johnson, Lemmens and Tijsterman, 2013). Day-1 adults were left laying eggs on seeded NGM plates for no longer than 1.5 h. Upon removal of the adult worms, early eggs were irradiated using an IR-inducing cesium-137 source.

### Alkylation damage induction by using methyl methanesulfonate treatment (MMS)

Synchronized L1 worms were incubated with different concentrations of MMS (Sigma, 129925) diluted in M9 buffer, moving for 1 hour at 20 °C. Worms were washed three times with M9 buffer, and plated in seeded NGM plates.

### Interstrand crosslink induction by using Cisplatin

Synchronized L1 worms were exposed to different concentrations of cisplatin in dimethyil-formamide (DMF) diluted in M9 buffer or mock-treated with DMF (Sigma, 227056) diluted M9 buffer for 2 hours at 20 °C. Afterwards, worms were washed three times with M9, allowed to grow for 48 h in NGM plates.

### Lifespan assay

Synchronized day-1 adult worms were irradiated or mock-irradiated with a 310 nm PL-L 36W/UV-B UV6 bulb (Waldmann, 451436623-00005077), then placed on fresh OP50-seeded NGM plates and incubated at 20 °C. The worms were transferred to new plates every other day to avoid progeny overgrowth. Worms presenting internal hatching or protruding or ruptured vulvas were censored, and death worms were scored when no movement or pumping was observed even upon physical stimulus.

### DNA repair capacity assay in L1 worms (Slot blot)

The quantification of DNA repair via immunostaining of CPDs of DNA samples in a slot blot was performed as previously described with slight changes (Rieckher *et al*., 2017). Briefly, bleach-synchronized L1 worms (at least 30.000 per plate) were irradiated with UV-B light and split in two groups, one to be immediately quick-frozen in liquid nitrogen, the control of unrepaired damaged, and the other one to be left in seeded plates for 24h at 20 °C to allow DNA repair to occur. After this, to avoid bacterial DNA contamination, worms were washed 5 times, incubated 2 hours to permit the removal of intestinal bacteria, washed another 5 times and finally quick-frozen.

DNA extraction was performed using the Gentra^{®} Puregene^{®} Tissue Kit (Qiagen, 158667) and the protocol for DNA Purification from Tissue. 500 µl Cell Lysis Solution, 2.5 µl Puregene Proteinase K, 2.5 µl RNase A Solution, 170 µl Protein Precipitation Solution, 500 µl isopropanol and 500 µl 70% ethanol were used. Cell Lysis Solution was directly added to the thawed sample and additional step with Proteinase K was performed, incubating at 55 °C overnight. DNA concentration was measured using the Qubit^{®} dsDNA HS Assay Kit (Invitrogen, Q32851). Serial dilutions of the DNA were denatured at 95 °C for 5 minutes and transferred onto a Hybond nylon membrane (Amersham, RPN119B) using a Convertible Filtration Manifold System (Life Technologies, 11055). DNA crosslinking to the membrane was performed by incubating at 80 °C for 2 hours. The membrane was blocked for 30 minutes in 3% milk/PBS-T (0.1%) at RT. The membrane was incubated overnight at 4 °C with anti-CPDs (Clone TDM-2, 1:10.000, Cosmo Bio, CAC-NM-DND-001), then washed three times with PBS-T (5 minutes at RT), and blocked for 30 minutes with 3% milk/PBS-T. The secondary antibody used was a goat anti-mouse AffiniPure peroxidase-conjugated secondary antibody (1:10.000, Jackson Immuno Research, 115-0.5-174), followed by three washes in PBS-T and incubation with ECL Prime (Amersham, RPN2232). The DNA lesions were visualized by using a Hyperfilm ECL (Amersham, 28906836).

Finally, in order to stain and quantify the total amount of DNA per sample, the membrane was incubated overnight at 4 °C in PBS with 1:10.000 SYBR^{™} Gold Nucleic Acid Stain (Invitrogen, S11494), then washed 5 times 10 minutes in PBS at RT, and imaged using a BIO-RAD Gel Dox XR+ Gel Documentation System (BIO-RAD, 1708195).

### Adult Somatic DNA repair assay (Immunofluorescence)

Synchronized day-1 adult worms were irradiated or mock-irradiated with a 310 nm UV-B light Philips UV6 bulb in a Waldmann UV236B irradiation device. Half of the worms were left in seeded NGM plates for 60 h at 20 °C to allow DNA repair, whereas the others were collected directly after the irradiation. After irradiation or the incubation time respectively, worms were picked and placed in a drop of M9 buffer on top of a HistoBond+ Adhesion Microscope Slides (Marienfeld, 0810461). Using two standard disposable hypodermic needles we cut the worms close to the head, and then placed a coverslip over the slide and placed it at -80 °C for at least 30 minutes. The coverslip was removed quickly to perform freezecracking (Duerr, 2013), that besides the cutting of the worm, would improve the penetrance of the antibodies. Worms were fixated by placing the slides at liquid methanol at -20°C for 10 minutes, then washed 5 minutes in PBS. 70 µl of 2M HCI was added for 30 minutes at RT to denature the DNA. Slides were washed three times with PBS, and blocked with 70 µl of 20% Fetal Bovine Serum (FBS) in PBS for 30 minutes at 37 °C. After washing the blocking solution, the slides were incubated with 70 µl of 1:10.000 anti-CPDs (Clone TDM-2, 1:3.000, Cosmo Bio, CAC-NM-DND-001) in PBS containing 5% FBS, at 4 °C, overnight in a humid chamber. After washing three times in PBS for 5 minutes each, 70 µl of secondary anti-mouse Alexa Fluor 488 (1:300, Invitrogen, A21202) in 5% FBS PBS was added for 30 minutes at 37 °C. The slides were washed three times for 5 minutes and mounted using 5 µl of Fluoromount-G^{™} with DAPI (Invitrogen, 00495952).

### Image quantification of heads

Image stacks of the heads of adult worms were analyzed using the microscopy image analysis software, Imaris (Oxford Instruments). Nuclei in the area anterior to the pharyngeal-intestinal valve were determined by using the DAPI staining and setting a threshold of size and intensity. False positive nuclei (due to bacteria in the pharynx) were manually discarded. CPDs signal was quantified by using the maximum spherical volume fitting inside each of the nuclei. The 2-way ANOVA to analyze the interaction effect between the strain and the time components was calculated on log10 transformed values with Python's pingouin v0.3.6.

### Cell culture and treatments

U2OS were cultured in DMEM, high glucose GlutaMAX Supplement, Pyruvate (Thermo Fisher Scientific, 31966047) with 10% fetal bovine serum (FBS; Biochrom GmbH, S0615) and 1% Penicillin-Streptomycin (Thermo Fisher Scientific, 15140112). Cells were kept at 37 °C in a 5% CO₂ incubator (Binder). Cell dissociation from the plates was performed with Accutase (Sigma, A6964). Cells were cultivated in FBS-free medium for 48 hours before genotoxic treatment were applied. 24 hours after FBS-free medium, cells were mock treated or received harmine hydrochloride (diluted in water) or INDY (diluted in DMSO) (Sigma, SMB00461 and SML1011) at 10 or 25 µM respectively. Before the genotoxic treatment, cells were washed with FBS-free medium. For the UV treatment, medium was removed from the plates and cells were irradiated using 254 nm UV-C light Phillips UV6 bulbs. The MMS treatment was performed by adding MMS at 2 mM for 2 hours, followed by 3 washes with FBS-free medium. Quantification via FACS of cell death and apoptosis was performed 24 hours after genotoxic treatment.

### FACS analysis

Collected cells were incubated in Annexin V Binding buffer (BioLegend, 422201) with Pacific Blue Annexin V (BioLegend, 640917) and 7-AAD (Thermo Fisher Scientific, 00699350) at 4 °C for 15 minutes. Cells were measured using a MACSQuant VYB (Miltenyi Biotec) and analyzed using FlowJo (BD).

### RNA extraction for RNA-seq and qPCR experiments

For the qPCRs and RNA-seq on L1 worms, around 10.000 (qPCR) or 40.000 (RNA-seq) bleach-synchronized L1 worms in triplicates (qPCR) or quadruplicates (RNA-seq) per strain and condition were placed in seeded NGM plates and left at 20 °C for 3 hours. Posteriorly, they were irradiated with a 310 nm UV-B light Philips UV6 bulb in a Waldmann UV236B irradiation device or left unirradiated, and left for 6 more hours to allow the DNA damage related transcriptional changes to be taking place. After this, worms were collected, washed three times with M9 buffer, and the pellet was placed in a tube containing 1 ml of TRIzol^{™} (Invitrogen, 15596018) and 1 mm zirconia/silica beads (Biospec Products, 11079110z). To extract the RNA, worms were first disrupted with a Precellys24 (Bertin Instruments, P000669-PR240-A), and then the RNA isolation was performed by using the RNeasy Mini Kit (QIAGEN, 74106) following the manufacturer's specifications, except for the use of 1-bromo-3-chloropropane (Sigma, B9673) instead of chloroform. The RNA was quantified using NanoDrop ^{™}8000 (ThermoFisher, ND-8000-GL). RNA extraction from U2OS cells was performed after 24 hours of harmine or INDY treatment of cells starved for a total of 48 hours, by using the RNeasy Mini Kit following the manufacturer's specifications. Cells were disrupted with RLT buffer and homogenized with QIAshredder spin columns (QIAGEN, 79656).

### qPCRs

Reverse transcription to form cDNA was performed using Superscript III (Invitrogen, 18080044). The obtained cDNA was used to perform qPCR by using SYBR Green I (Sigma, S9460) and Platinum Taq polymerase (Invitrogen, 10966034) in a BIO-RAD CFX96 real-time PCR machine (BIO-RAD, 1855196). The analysis of the results was performed by using the comparative C_{T} method(Schmittgen and Livak, 2008).

All *C. elegans* qPCR experiments were done in biological triplicates and the data was normalized to three housekeeping genes.

**Table 2: C. elegans qPCR PRIMERS**

| **Housekeeping genes:** | **Forward primer** | **Reverse primer** |
|---|---|---|
| *Y45F10D.4* | SEQ ID NO 1: | SEQ ID NO 2: |
| | CGAGAACCCGCGAAATGTCGGA | CGGTTGCCAGGGAAGAT- GAGGC |
| *eif-3.C* | SEQ ID NO 3: | SEQ ID NO 4: |
| | ACACTTGACGAGCCCACCGAC | TGCCGCTCGTTCCTTCCTGG |
| *vha-6* | SEQ ID NO 5: | SEQ ID NO 6: |
| | CTGCTATGTCAATCTCGG | CGGTTACAAATTTCAACTCC |

| **Genes of interest:** | | |
|---|---|---|
| *parp-1* | SEQ ID NO 7: | SEQ ID NO 8: |
| | AGCGAATGAAGAAACAATCCGA | |
| | | _ACTAGGCGTTCGATTACTT- GTG |
| *polh-1* | SEQ ID NO 9: | SEQ ID NO 10: |
| | AGAAATATCGCGACGCTAGC | GTAGGTAATAGCAGCCTGCA |
| *polk-1* | SEQ ID NO 11: | SEQ ID NO 12: |
| | GAGATACTGATGGAGAATCTT- GAG | AGTAGTTGGATGTGCTCAGC |
| *mus-101* | SEQ ID NO 13: | SEQ ID NO 14: |
| | TCGAAAGCCATATACGAT- GAACC | ACAAGAACGGGAGTACTAG- AGAC |
| *exo-3* | SEQ ID NO 15: | SEQ ID NO 16: |
| | GGAGGAGACGTT- TAAGAACTACAC | TAGATCACTGGCTTCTTCTCGT |
| *lig-1* | SEQ ID NO 17: | SEQ ID NO 18: |
| | TGATCAAGGCTGTTGCTAAAGC | AGCCTCAATTCCTTGACATGC |
| *atm-1* | SEQ ID NO 19: | SEQ ID NO 20: |
| | GCGAAGTTCTTACACCTCGAC | AGTTCGACACATTCTTCAGCA |

U2OS qPCR experiments were done using six biological replicates and the data was normalized to HPRT and GPDH.

**Table 3: U2OS qPCR PRIMERS**

| **Housekeeping genes:** | **Forward primer** | **Reverse primer** |
|---|---|---|
| HPRT | SEQ ID NO 21: | SEQ ID NO 22: |
| | GACCAGTCAACAGGGGACAT | CCTGACCAAGGAAAGCAAAG |
| GPDH | SEQ ID NO 23: | SEQ ID NO 24: |
| | GACCAGTCAACAGGGGACAT | TTAAAAGCAGCCCTGGTGAC |

| **Genes of interest:** | | |
|---|---|---|
| CCNB1 | SEQ ID NO 25: | SEQ ID NO 26: |
| | GATTGGAGAGGTTGATGTCGAG | AGTCATGTGCTTTGTAAGTCCT |
| FEN1 | SEQ ID NO 27: | SEQ ID NO 28: |
| | AAAGGCCAGTCATCCCTCC | TTGCCATCAAAGACATACACGG |
| PLK1 | SEQ ID NO 29: | SEQ ID NO 30: |
| | TATTCCCAAGCACATCAACC | TAGCCAGAAGTAAAGAACTCGT |
| POLQ | SEQ ID NO 31: | SEQ ID NO 32: |
| | GCAACTTCTACTCTTTCTTCTGG | ATACTCTCGCCTACTGTGTC |
| RAD21 | SEQ ID NO 33: | SEQ ID NO 34: |
| | ACAGACTACTGAAGCTCTTTA- CAC | GGGCTCATCGATAACATCAC |
| RAD51 | SEQ ID NO 35: | SEQ ID NO 36: |
| | GTTCAACACAGACCACCAGAC | CTACACCAAACTCATCAGCGA |

### RNA-seq

A triplicate of RNA samples from *lin-52* mutant and WT L1 worms were rRNA depleted using Ribo-Zero Plus rRNA Depletion Kit (Illumina, 20037135) and sequenced using a Hiseq4000 (Illumina) with PE75 read length. RNA quality control showed RIN ≥ 9.4 for all samples. The RNA-seq data were processed through the QuickNGS pipeline (Wagle, Nikolić and Frommolt, 2015), Ensembl version 85. Reads were mapped to the *C. elegans* genome using Tophat (Kim *et al*., 2013) (version 2.0.10) and abundance estimation was done using with Cufflinks (Trapnell *et al*., 2010) (Version 2.1.1). DESeq2 (Love, Huber and Anders, 2014)was used for differential gene expression analysis.

The human RNA-seq data were processed with Salmon-1.1 (Patro *et al*., 2017) against a decoy-aware transcriptome (gencode.v37 transcripts and the GRCh38.primary_assembly genome) with the following parameter: --validateMappings -gcBias -seqBias. The output was imported and summarized to the gene-level with tximport (1.14.2) (Soneson, Love and Robinson, 2016) and differential gene analysis was done with edgeR (3.28.1) (Robinson, McCarthy and Smyth, 2009).

### Datasets

The list of 211 genes belonging to the GO term "Cellular response to DNA damage stimulus" was obtained by using data from the Gene Ontology Consortium(Ashburner *et al*., 2000; Carbon *et al.*, 2019) (database released on 2019-10-08). Gene IDs from previously published datasets were updated to current databases, and duplicated or dead IDs were eliminated accordingly. Obtention of updated gene IDs and transformation of names to Wormbase IDs or Ensembl IDs was done with WormBase's SimpleMine(Harris *et al*., 2020) and Ensembl's BIOMART (Yates *et al.*, 2020) respectively. Overlap analysis were done by using Fisher's exact test in R.

### Data access

The *C*. *elegans* RNA-seq data used in this study are available from Gene Expression Omnibus (GEO; http://www.ncbi.nlm.nih.goz/geo) with the accession number GSE152235 and secure token yvqvewuetzkjrsl. The human RNA-seq data is available with the accession number GSE168401 and secure token wjehcacaflkxrip.

### Analysis of DNA repair kinetics in human U2OS cells upon addition of an inhibitor of DREAM complex assembly

Cells are cultured as described above. Next, cells are mock treated or receive harmine hydrochloride (diluted in water) or INDY (diluted in DMSO) (Sigma, SMB00461 and SML1011) at 10 or 25 µM respectively. Before the genotoxic treatment, cells are washed with FBS-free medium. For the UV treatment, medium is removed from the plates and cells are irradiated using 254 nm UV-C light Phillips UV6 bulbs. The MMS treatment is performed, e.g., by adding MMS at 2 mM for 2 hours, followed by 3 washes with FBS-free medium. Cells are fixed in a solution comprising PFA on HistoBond+ Adhesion Microscope Slides (Marienfeld, 0810461). To estimate the total amount of DNA damage per cell, the fixed cells are incubated overnight at 4 °C in PBS comprising anti-yH2AX antibody (Mouse anti-phospho-Histone H2A.X (Ser139), clone JBW301, Millipore, Cat#: 05-636), then washed 5 times 10 minutes in a suitable buffer at RT, prior to incubation for at least two hours with a secondary antibody. Finally, the slides are washed another 5 times 10 minutes in PBS-T and dried overnight. The next morning, slides are sealed with a cover slip.

### Results

### DNA damage response gene promoters carry the CDE-CHR DREAM binding motif

We assessed whether specific transcription factor binding sites might be overrepresented in promoters of DDR genes. We performed an unbiased DNA motif enrichment analysis of the 211 DDR genes in *C*. *elegans,* based on the Gene Ontology Consortium classifications (Ashburner *et al*., 2000; Carbon *et al*., 2019). This analysis revealed a significant enrichment of the DPL-1, EFL-1 and LIN-15B binding motifs in the DDR gene promoters (Fig. 1A). DPL-1 and EFL-1 form the E2F-DP heterodimer that directly contacts promoters by binding to the cycle-dependent element (CDE). E2F-DP is linked via the pocket protein LIN-35 to the MuvB subcomplex, which binds the cell cycle genes homology region (CHR) in promoters to then, together, form the DREAM transcriptional repressor complex (Muller and Engeland, 2010; Müller *et al*., 2012; Goetsch, Garrigues and Strome, 2017). To address whether the DREAM complex target sites were present in the DDR genes, we queried whether the CDE-CHR motif was overrepresented. Strikingly, a total of 125 of the 211 DDR genes contained the CDE-CHR motif in the promoter area (Fig. 1B, one mismatch allowed, 0 to -1000 bp before transcription start site; p-value = 5.16e-07), which suggested that the DREAM complex might be a regulator of DDR genes.

### Loss-of-function mutations of DREAM complex components confer resistance to UV-induced DNA damage

We next determined whether mutations in DREAM components might influence DNA damage sensitivity. UV-B irradiation induces cyclobutane pyrimidine dimers (CPDs) and pyrimidine (6-4) pyrimidone photoproducts (6-4PPs) that are repaired by NER. We exposed synchronized L1 larvae carrying mutations in the DREAM complex to different doses of UV-B. 48 h post UV, the developmental growth was scored by counting the stages from L1 to the consecutive L2, L3, L4 larval and adult stages. The DREAM mutants *lin-52*, *dpl-1*, *efl-1*, *lin-53, and lin-35* showed a significant improvement in proceeding through somatic development compared to wild-type (WT) worms following UV exposure (Fig. 1C). *lin-52; dpl-1* double mutants that carry a defect in each of the two subcomplexes that form the DREAM showed a similarly improved developmental growth as the respective single mutants, indicating that their role as DREAM complex subunits conferred the UV resistance (Fig. 1D). Thus, DREAM mutants confer resistance to UV-induced DNA damage during development, indicating that the specific function of *lin-52, dpl-1, efl-1, lin-53* and *lin-35* as subunits of the DREAM complex determine the animals' ability to overcome DNA damage-induced developmental impairments.

To evaluate whether mutations in the DREAM complex could affect DNA damage-driven organismal aging, we UV-treated the DREAM complex mutant worms on day 1 of adulthood and assessed their lifespan (Fig. 1E). While in humans, mutations in DNA repair genes are sufficient to accelerate aging and lead to premature death (da Silva and Schumacher, 2019), *C. elegans* cultured under laboratory conditions requires exogenous DNA damage to shorten lifespan (Lans *et al.,* 2013; Mueller *et al.,* 2014; Bianco and Schumacher, 2018). UV irradiation led to a pronounced decrease in lifespan, which was significantly milder in all the DREAM complex mutants tested. *lin52, dpl-1, efl-1* and *lin-35* mutants significantly outlived wild-type worms upon DNA damage, despite the fact that some were short-lived without irradiation.

In addition to survival, we also assessed the motility of the worms as an important parameter of the maintenance of organismal health (Keith *et al.,* 2014). Upon UV treatment, *lin-52* mutants retained motility to a greater extent than WT animals, indicative of improved organismal health upon DNA damage. Overall, due to the phenotypic specificity with, comparatively, mild adverse effects under unperturbed conditions and the strong UV-resistance phenotype, we decided to focus mainly on *lin-52* mutants to further investigate the role of the DREAM complex in regulating genome stability from here onwards.

### DREAM complex mutants improve repair of UV-induced DNA lesions

We next tested whether the UV resistance of the DREAM complex mutants might be a consequence of enhanced DNA repair activity. We measured the worms' capacity to repair cyclobutane pyrimidine dimers (CPDs), which is the main UV-induced DNA lesion type that can be detected by an established and highly specific anti-CPD antibody (Fig. 2). We irradiated L1 larvae and quantified the removal of CPDs after 24 h using an anti-CPD antibody in a DNA slot blot. The DREAM complex mutants tested showed a significantly improved CPD repair compared to WT, whereas, as expected, a mutation in the NER core component xpa-1, showed a diminished CPD removal (Fig. 2A). To exclude that the enhanced removal of CPDs might be a consequence of CPD dilution due to overall DNA replication, we performed an EdU incorporation assay in L1 worms upon the same dose of UV irradiation. *lin-*52 mutants and WT worms showed comparable DNA replication events regardless of UV irradiation. Thus the decreased amount of CPDs was a consequence of improved repair capacity.

We next assessed whether the DREAM complex also regulates the DNA repair capacity in adult animals. Day 1 adult worms were UV-irradiated and the CPDs were quantified by anti-CPD antibody staining *in situ* (Fig. 2B). We focused on the head of the worm because of the high density of somatic nuclei in this region. Similarly, as in the case of L1 larvae, DREAM complex mutants showed an improved removal of CPD lesions compared to WT worms indicating an augmented repair capacity.

NER is initiated either by the TC-NER protein CSB-1 or by the GG-NER protein XPC-1 (Lans *et al.,* 2010). We next evaluated whether mutations in the DREAM complex required one of these branches to confer the improved resistance to UV-induced DNA damage. The *lin-52* mutation alleviated the UV sensitivity of *csb-1, csa-1,* and *xpc-1* mutants. Consistent with the requirement for NER for removing UV lesions, the mutant *lin-52* had no effect on completely NER deficient *xpa-1* or *csb-1; xpc-1* double mutants. Therefore, a mutation in the DREAM complex improved both GG-NER and TC-NER and could thus partially compensate for defects in either one of the NER initiating systems, while the enhanced UV resistance depends on the presence of the NER machinery.

### The DREAM complex represses multiple DNA repair genes and pathways

We hypothesized that the DREAM complex could be directly regulating genes involved in the DNA repair process, thus curbing the repair capacity of somatic cells. We performed RNA-sequencing of *lin-52(n771)* and WT L1 larvae (Fig. 3). The majority of differentially expressed genes were upregulated in *lin-52* mutants and these genes included a range of genes involved in DNA repair mechanisms (Fig. 3A and Tab. 4). Gene Ontology (GO) analysis of the significantly upregulated genes (p-adj < 0.05) in *lin-52* mutants compared to WT showed that the terms "Cellular response to DNA Damage stimulus", "DNA repair" and "DNA metabolic process" were highly enriched (FDR corrected p-values of 1.23e-27, 1.10e-25 and 8.42e-31, respectively). We confirmed the upregulation of DNA repair genes by qPCR analysis, and also determined that DDR genes were upregulated in other DREAM complex mutants (Fig. 3B). Thus, the DREAM complex represses the expression of genes involved in DNA repair mechanisms. A total of 53 genes involved in cellular responses to DNA damage were significantly upregulated (p-adj < 0.05) in *lin-52* mutants compared to WT worms (Tab. 4). Among these 53 genes, there are genes involved in NER, interstrand crosslink (ICL) repair, base excision repair (BER), HRR, mismatch repair (MMR), and NHEJ, suggesting that the DREAM complex represses components of all the main DNA repair pathways (Fig. 3D).

We next asked whether the DNA repair genes we found de-repressed in *lin-52* mutants, and confirmed in *efl-1* and *dpl-1* mutants (Fig. 3B), would also be present in other DREAM complex transcriptomic data. The induced DNA repair genes in *lin-52* mutants showed a remarkably consistent induction in two transcriptome datasets on *lin-35(n745)* mutants (Latorre *et al.,* 2015; Sierra *et al.,* 2015). We find that 52 DDR genes were upregulated in *lin-35* L1s and 62 in *lin-35* L3 worms. Furthermore, we observe a striking overlap between these up-regulated DDR genes and those found in *lin-52.* Out of the 53 DDR genes up-regulated in *lin-52,* 35 were common with *lin-35* L1s, and 44 with *lin-35* L3 worms (Fig. 3D and Tab. 4). The high amount and overlap of the DDR genes regulated by *lin-52* and by *lin-35* mutants in two independent studies together with the qPCR results in mutants for *dpl-1* and *efl-1* (Fig. 3C) indicate that the DREAM complex regulates an extensive amount of DDR genes.

To address whether the induced DDR genes might be directly repressed by the DREAM complex, we analyzed a published ChIP-seq dataset on the DREAM complex from late embryos (Goetsch, Garrigues and Strome, 2017). 43 out of the 53 DDR genes upregulated in *lin-52* mutants are bound by the DREAM complex (41 in the promoter area, 2 intergenic or intronic), i.e. by at least 6 out of 7 DREAM components tested (Goetsch, Garrigues and Strome, 2017) (Fisher's Exact *p*-value < 2.2e-16) (Fig. 3E and Tab. 4). We confirmed the direct binding of DREAM to DDR genes by analyzing a ModENCODE ChIP seq dataset for DREAM in L3-stage worms (Latorre *et al.,* 2015). 34 of the 53 DDR related genes that were upregulated in *lin-52* mutants were bound by at least one member of the DREAM complex and 17 by all of them (one not found), a highly significant enrichment (Fisher's Exact p-value < 2.2e-16 and 8.348e-14, respectively).

We analyzed whether among all the genes classified as cellular responses to DNA damage, there are more directly bound by the DREAM complex beyond our findings with *lin-52.* Using the embryonic ChIP-seq data (Goetsch, Garrigues and Strome, 2017), we found that 80 out of 211 DDR genes were bound by the DREAM complex (76 in the promoter, 4 intergenic/intronic, Fig. 3E). This overlap was highly significant (Fisher's Exact p-value < 2.2e-16). A similar result was obtained when analyzing the ModENCODE L3-stage data (Latorre *et al.,* 2015): 200 out of 211 genes had data available, of which 61 were bound by at least one component of the DREAM complex (Fisher's Exact p-value < 2.2e-16). These analyses reveal that the DREAM complex directly binds and represses multiple genes involved in the DDR. The consistency of the induction of DDR genes in DREAM mutants and the direct binding of DREAM components to DDR gene promoters across studies using embryos and different larval stages indicate that DREAM constitutively represses DDR genes in somatic cells. In conclusion, our analysis of RNA-seq and ChIP-seq data indicates that the DREAM complex constitutively represses DDR genes operating in all major DNA repair mechanisms in somatic cells.

**Table 4: The DREAM complex binds and represses DNA damage response (DDR) gene expression. Induction of the expression of DDR gene in lin-52 mutants is highly consistent in lin-35 mutants at L1 (Kirienko and Fay, 2007) and L3 stage (Latorre et al., 2015) and promoters of these genes are bound by DREAM (re-analysis of embryonic ChIP-seq data (Goetsch et al., 2017)). DDR list based on the GO databased released on 2019-10-08. p-adj = p-value with Benjamini-Hochberg (FDR) adjustment, FC = Fold Change, NA = not applicable due to the gene information not being available or in the dataset, in brackets = the FC was non-significant, ✔ = bound by at least 6 DREAM components in the promoter, ✔* = bound by at least 6 DREAM components in intronic or intergenic areas.**

| Gene | FC *lin-52* vs WT | *p*-adj | FC *lin-35* vs WT (L1) | FC *lin-35* vs WT (L3) | Bound by DREAM |
|---|---|---|---|---|---|
| *atm-1* | 1.53 | 1.91e-53 | NA | 1.52 | ✔ |
| *baf-1* | 1.15 | 2.96e-03 | NA | 1.35 | ✔ |
| *brc-1* | 1.34 | 2.39e-06 | 1.95 | 2.57 | ✔ |
| *brd-1* | 1.23 | 9.11e-03 | 1.78 | 2.39 | ✔ |
| *chk-1* | 1.18 | 1.39e-02 | 2.18 | 1.73 | ✔ |
| *cku-80* | 1.32 | 1.63e-08 | 2.43 | 2.31 | ✔ |
| *clsp-1* | 1.21 | 6.22e-05 | 2.27 | 2.59 | ✔ |
| *crn-1* | 1.30 | 7.34e-11 | 1.57 | 1.96 | ✔ |
| *csa-1* | 1.38 | 1.15e-04 | 4.37 | 4.87 | ✔ |
| *ctf-4* | 1.16 | 2.23e-03 | 2.07 | 1.70 | ✔ |
| dog-1 | 1.27 | 1.74e-05 | 1.76 | 2.23 | ✔ |
| exo-1 | 1.27 | 4.24e-05 | 2.64 | 2.43 | ✔ |
| *exo-3* | 2.08 | 4.70e-37 | 2.99 | 2.07 | ✔ |
| *F10C2.4* | 1.35 | 3.99e-19 | 1.62 | 1.22 | ✔ * |
| *fan-1* | 1.32 | 5.34e-05 | 1.79 | 1.55 | ✔ |
| *fcd-2* | 1.14 | 9.27e-03 | NA | [1.16] | ✔ |
| *H21P03.2* | 1.34 | 2.97e-06 | 1.61 | 1.63 | ✔ |
| *him-1* | 1.10 | 8.10e-03 | NA | [1.10] | ✔ |
| *his-3* | 1.24 | 2.14e-23 | NA | NA | ✔ |
| *hmg-12* | 1.74 | 1.50e-39 | 1.87 | [1.16] | ✔ |
| *hpr-17* | 1.31 | 2.18e-03 | 2.17 | 1.93 | ✔ |
| *hsr-9* | 1.18 | 2.03e-09 | NA | [1.15] | ✔ |
| *JC8.7* | 1.24 | 1.43e-03 | NA | [1.21] | ✔ |
| *lig-1* | 1.65 | 2.37e-42 | 2.93 | 2.66 | ✔ |
| *M03C11.8* | 1.37 | 5.61 e-30 | NA | 1.26 | ✔ |
| *mcm-3* | 1.19 | 3.23e-06 | 1.80 | 2.06 | ✔ |
| *mcm-4* | 1.11 | 7.38e-04 | 1.63 | 1.63 | ✔ |
| *mcm-6* | 1.12 | 1.06e-03 | NA | 1.40 | ✔ |
| *mcm-7* | 1.20 | 7.23e-10 | 2.53 | 2.59 | ✔ |
| *mre-11* | 1.21 | 1.63e-04 | 2.15 | 1.67 | ✔ |
| *mrt-1* | 1.58 | 7.63e-10 | 2.32 | 2.48 | ✔ |
| *msh-6* | 1.25 | 2.43e-10 | 2.62 | 2.12 | ✔ |
| *mus-101* | 1.87 | 7.35e-72 | 4.13 | 2.51 | ✔* |
| *parg-1* | 1.45 | 5.98e-32 | 1.66 | 1.89 | ✔ |
| *parp-1* | 2.35 | 5.46e-85 | 3.36 | 3.27 | ✔ |
| *pms-2* | 1.29 | 2.58e-03 | NA | 1.35 | ✔ |
| *polh-1* | 2.14 | 6.85e-45 | 3.40 | 3.57 | ✔ |
| *polk-1* | 1.63 | 4.18e-15 | 2.75 | 3.18 | ✔ |
| *rad-50* | 1.51 | 1.05e-30 | 2.11 | 1.35 | ✔ |
| *rad-51* | 1.27 | 3.44e-04 | 1.86 | 1.97 | ✔ |
| *rad-54* | 1.48 | 8.45e-09 | NA | 1.60 | ✔ |
| *rnf-113* | 1.23 | 3.15e-04 | NA | 1.29 | ✔ |
| *rpa-1* | 1.08 | 2.43e-02 | NA | 1.25 | ✔ |
| *ruvb-1* | 1.13 | 9.05e-03 | NA | [0.92] | ✔ |
| *smc-3* | 1.20 | 4.95e-10 | NA | 1.38 | ✔ |
| *smc-5* | 1.42 | 4.82e-18 | 2.20 | 1.79 | ✔ |
| *smc-6* | 1.15 | 1.32e-03 | 1.56 | [1.14] | ✔ |
| *sws-1* | 1.35 | 2.06e-04 | 3.93 | 3.37 | ✔ |
| *tdpt-1* | 1.30 | 2.85e-04 | NA | 1.86 | ✔ |
| *tim-1* | 1.27 | 2.36E-13 | 1.97 | 2.04 | |
| *tipn-1* | 1.70 | 2.31e-22 | 5.34 | 6.98 | |
| *trr-1* | 1.12 | 1.94e-07 | NA | [1.02] | |
| *ung-1* | 1.37 | 6.70e-05 | NA | 1.90 | |

### DREAM mutations confer resistance to a wide variety of DNA damage types

Given that we found a range of DNA repair pathways induced in the soma of *lin-52* mutants, we hypothesized that DREAM complex mutants would show a resistance phenotype to a wide variety of DNA damaging insults that require different repair machineries for their removal. We first evaluated how early embryos would be affected by the induction of double-strand breaks (DSBs) by ionizing radiation (IR). Somatic cells in the early embryo are highly replicative and only during this early stage employ HRR, which is initiated by the BRC-1/BRD-1 complex (Clejan, Boerckel and Ahmed, 2006; Johnson, Lemmens and Tijsterman, 2013; Janisiw *et al.,* 2018). Upon egg-laying of day 1 adults, eggs were exposed to different IR doses. 24 h later the number of hatched larvae was evaluated for WT, *lin-52(n771),* the HRR deficient *brc-1(tm1145); brd-1(dw1)* double mutant and the *lin-52; brc-1; brd-1* triple mutant (Fig. 4A). The proportion of egg hatching upon IR in *lin-52(n771)* was significantly higher than in WT worms, and as expected, *brc-1; brd-1* deficient eggs were highly IR sensitive. Surprisingly, mutant *lin-52* rescued the IR sensitivity of *brc-1; brd-1* double mutants to levels even above that of WT animals, suggesting that in the absence of HRR, a mutation in *lin-52* can induce alternative DSB repair pathways that are able to compensate for the lack of HRR. The IR resistance conferred by mutant *lin-52* in early embryos, which are comprised of the most rapidly proliferating cells in *C. elegans,* indicates that the repression of DNA repair genes is a property of a somatic function of the DREAM complex and not just associated with cellular quiescence or terminal differentiation.

We wished to know whether a deficiency in the DREAM complex would also render the worms resistant to DSBs in a NHEJ repair-dependent fashion. WT, *lin-52(n771),* the NHEJ deficient mutant *cku-70(tm1524)* and the double mutant *lin-52; cku-70* L1 larvae were exposed to IR and their developmental growth assessed 48 h later. IR-treated *lin-52* mutants showed a significantly improved developmental growth compared to WT worms. However, this enhanced repair was dependent on NHEJ, as *lin-52* could not alleviate the sensitivity to IR of *cku-70* mutants (Fig. 4B). We also analyzed two other strains that are sensitive to IR (Roerink *et al.,* 2012), *xpa-1(ok698)* and *polh-1(lf31),* and observed a strong sensitivity to IR during development, but when crossed with mutants for *lin-52* this sensitivity was significantly rescued for both mutants. These results indicate that mutations in *lin-52* enhance the NHEJ-dependent DSB repair, resulting in augmented IR resistance in IR-sensitive strains that have the canonical NHEJ pathway intact.

We evaluated how a mutation in the DREAM complex would respond to alkylation damage, a complex DNA insult known to be repaired by several mechanisms involving DNA methyltransferases, AlkB enzymes and BER (Soll, Sobol and Mosammaparast, 2017). L1 worms were exposed to an acute dose of methyl methanesulfonate (MMS) and their development was assessed 48 h later. We employed WT, *lin-52(n771),* and *polh-1(lf31),* and the double mutant *lin-52; polh-1* (Fig. 4C). The mutation in the DREAM complex component *lin-52* led to an improved development following MMS treatment and suppressed the MMS sensitivity of *polh-1* mutant animals to levels comparable to the one of WT worms.

We assessed the response to cisplatin, a commonly used antitumor drug known to cause intra- and inter-strand crosslinks (Deans and West, 2011). L1 larvae were treated with increasing doses of cisplatin and the development of WT and *lin-52(n771)* was evaluated 48 h later (Fig. 4D). A mutation in the DREAM complex significantly alleviated the growth retardation upon cisplatin induced DNA-damage for all doses tested.

Taken together, these data show that *lin-52* mutants are resistant to a wide array of DNA damage types and alleviate DNA damage sensitivity of various mutants in single repair systems.

### Inhibition of the DREAM complex kinase DYRK1A confers DNA damage resistance in human cells and boosts DNA repair activity

Based on our *C. elegans* findings, we next wondered whether inhibition of the highly conserved DREAM complex could provide a pharmacological approach to augment DNA repair capacities in human cells. We queried whether similarly to nematodes, the human DREAM complex might also bind DDR gene promoters. We analyzed ChIP-seq data from quiescent human cells (Litovchick *et al.,* 2007) and searched for bound DNA repair genes following a similar criteria than the analyzed dataset from *C*. *elegans* (Goetsch and Strome, 2019) (Fig. 3E) and our promoter analysis (Fig. 1A, B). We selected genes bound by at least LIN9, p130 and E2F4 simultaneously, where the binding occurred in 5' between 0 and -1000 bp of the TSS. Among the 328 genes whose promoters were bound by DREAM, 67 genes are GO-classified as "DNA repair" (significantly overrepresented, Fisher's Exact p-value < 2.2e-16, Fig. 5A). Thus, the DREAM transcription repressor complex directly binds to promoters of DNA repair genes indicating that the DREAM-mediated DNA repair gene regulation is highly conserved in *C*. *elegans* and humans.

In mammals, DREAM components not only form the DREAM repressor complex but can also associate in other complexes that, unlike the DREAM complex, induce transcription (Osterloh *et al.,* 2007; Mannefeld, Klassen and Gaubatz, 2009; Sadasivam, Duan and DeCaprio, 2012). We, therefore, decided to use chemical inhibitors of the DYRK1A kinase, which phosphorylates LIN52, a modification required for the assembly of the DREAM complex thus allowing its specific abrogation (Litovchick *et al.,* 2011). We employed two potent but distinct chemical inhibitors of the DYRK1A kinase: The beta-carboline alkaloid harmine that has been widely used as specific DYRK1A inhibitor (Gockler *et al.,* 2009) but could also inhibit monoamine oxidase A (MAOA) and the benzothiazole derivative INDY that was established as a highly selective DYRK1A inhibitor (Ogawa *et al.,* 2010). As the DREAM complex represses gene expression in G0 cells, we serum starved confluent U2OS cells to obtain quiescent cell populations.

To confirm that the DYRK1A inhibitors abrogated DREAM-mediated gene repression, we performed RNAseq analysis of quiescent cells treated with either harmine hydrochloride or INDY. Among the significantly upregulated genes (FDR adjusted p-value < 0.01) all the known motifs bound by DREAM in human cells (Litovchick *et al.,* 2007) were significantly overrepresented, thus substantiating that INDY and harmine resulted in gene upregulation by inhibiting the DREAM complex. To confirm that the DYRK1A inhibitors lead to a similar derepression of DREAM targets, we plotted all the genes bound by the DREAM complex (Fig. 5A and Table 2) that were significantly up- or down-regulated (270 out of 328 genes with FDR corrected p-value < 0.01) upon harmine or INDY treatment (Fig. 5B). Most of the DREAM target genes were upregulated upon both treatments (corrected binomial test for the upper right quadrant p-value = 9.56e-33). Of the 67 DNA repair genes bound by DREAM (Figure 5A), 58 were upregulated upon harmine and 46 upon INDY treatment, 45 of which were upregulated in with both treatments (highlighted with orange, Fig. 5B). These results indicate that the pharmacological inhibition of DYRK1A with harmine or INDY results in upregulation of DREAM target genes, including the majority of DREAM targets encoding DNA repair genes.

To directly assess whether DYRK1A inhibitor treatment could augment DNA damage resistance, we exposed harmine- or INDY-treated quiescent cells to UV irradiation. We measured the apoptotic response to the DNA damage using Annexin V and 7-AAD analysis by FACS (Fig. 5C). Strikingly, both DYRK1A inhibitor treatments resulted in a highly significant reduction in UV-induced apoptosis compared to mock treated cells. As the DREAM complex targets multiple DNA repair genes both in nematodes and humans (Fig. 3 and 5A), we hypothesized that the harmine and INDY treated cells could be used to elevate resistance also to other DNA damage types. Consistently, DYRK1A inhibitor treatment boosted the resistance to the alkylating agent MMS (Fig. 5D). In conclusion, pharmacological inhibition of the DREAM complex kinase DYRK1A increased the expression of DREAM target genes involved in DNA repair and conferred resistance to distinct types of DNA damage, suggesting a highly conserved function of the DREAM complex in regulating DNA repair capacities.

To analyze whether DYRK1A inhibitor treatment augments DNA repair in quiescent cells, harmine- or INDY-treated quiescent cells are exposed to UV irradiation followed by antibody staining against the well-known DNA damage response marker γH2AX. In line with the results obtained from the CPD-staining performed in *C*. *elegans,* human quiescent cells that are treated with the DYRK1A inhibitors are expected to exhibit significantly lower amounts of yH2AX hours after UV-irradiation as compared to control cells that receive the UV treatment but are not exposed to either harmine or INDY, and, thus, that UV-induced lesions are repaired more readily in quiescent cells upon inhibition of DREAM assembly.

**Table 5. DREAM binds DDR genes in the promoters of quiescent cells.**

| List including all the genes bound in the promoter (0 to -1000 bp) by at least three components of DREAM (re-analysis of reference (Litovchick *et al*., 2007)),among them, the 67 genes involved in DNA repair ("✔", database released 2021-01-01), of which 58 and 46 we identified as upregulated upon harmine or INDY treatments respectively ("✔"). | | | | |
|---|---|---|---|---|
| **Gene stable ID** | **Gene name** | **DNA repair genes** | **Harmine DNA repair UP** | **INDY DNA repair UP** |
| ENSG00000004700 | RECQL | ✔ | ✔ | ✔ |
| ENSG00000013573 | DDX11 | ✔ | ✔ | ✔ |
| ENSG00000051180 | RAD51 | ✔ | ✔ | ✔ |
| ENSG00000051341 | POLQ | ✔ | ✔ | ✔ |
| ENSG00000062822 | POLD1 | ✔ | ✔ | ✔ |
| ENSG00000071539 | TRIP13 | ✔ | ✔ | ✔ |
| ENSG00000072501 | SMC1A | ✔ | ✔ | ✔ |
| ENSG00000073111 | MCM2 | ✔ | ✔ | ✔ |
| ENSG00000077152 | UBE2T | ✔ | ✔ | |
| ENSG00000080345 | RIF1 | ✔ | ✔ | ✔ |
| ENSG00000083093 | PALB2 | ✔ | ✔ | ✔ |
| ENSG00000085999 | RAD54L | ✔ | ✔ | ✔ |
| ENSG00000092853 | CLSPN | ✔ | ✔ | ✔ |
| ENSG00000097046 | CDC7 | ✔ | ✔ | ✔ |
| ENSG00000100479 | POLE2 | ✔ | ✔ | ✔ |
| ENSG00000104889 | RNASEH2A | ✔ | ✔ | ✔ |
| ENSG00000106268 | NUDT1 | ✔ | ✔ | ✔ |
| ENSG00000108384 | RAD51C | ✔ | ✔ | ✔ |
| ENSG00000109674 | NEIL3 | ✔ | ✔ | ✔ |
| ENSG00000111206 | FOXM1 | ✔ | ✔ | ✔ |
| ENSG00000111602 | TIMELESS | ✔ | ✔ | ✔ |
| ENSG00000117748 | RPA2 | ✔ | ✔ | |
| ENSG00000121988 | ZRANB3 | ✔ | ✔ | |
| ENSG00000123374 | CDK2 | ✔ | ✔ | ✔ |
| ENSG00000125871 | MGME1 | ✔ | ✔ | ✔ |
| ENSG00000125885 | MCM8 | ✔ | ✔ | |
| ENSG00000132646 | PCNA | ✔ | ✔ | ✔ |
| ENSG00000132781 | MUTYH | ✔ | ✔ | ✔ |
| ENSG00000133119 | RFC3 | ✔ | ✔ | ✔ |
| ENSG00000134574 | DDB2 | ✔ | ✔ | |
| ENSG00000136492 | BRIP1 | ✔ | ✔ | |
| ENSG00000138376 | BARD1 | ✔ | ✔ | |
| ENSG00000139618 | BRCA2 | ✔ | ✔ | ✔ |
| ENSG00000141499 | WRAP53 | ✔ | ✔ | ✔ |
| ENSG00000146263 | MMS22L | ✔ | ✔ | |
| ENSG00000146670 | CDCA5 | ✔ | ✔ | ✔ |
| ENSG00000147536 | GINS4 | ✔ | ✔ | ✔ |
| ENSG00000152422 | XRCC4 | ✔ | ✔ | |
| ENSG00000154920 | EME1 | ✔ | ✔ | ✔ |
| ENSG00000162607 | USP1 | ✔ | ✔ | ✔ |
| ENSG00000163918 | RFC4 | ✔ | ✔ | ✔ |
| ENSG00000164611 | PTTG1 | ✔ | ✔ | ✔ |
| ENSG00000164754 | RAD21 | ✔ | ✔ | ✔ |
| ENSG00000166801 | FAM111A | ✔ | ✔ | |
| ENSG00000168496 | FEN1 | ✔ | ✔ | |
| ENSG00000175279 | CENPS | ✔ | ✔ | ✔ |
| ENSG00000178295 | GEN1 | ✔ | ✔ | ✔ |
| ENSG00000178966 | RMI1 | ✔ | ✔ | ✔ |
| ENSG00000182481 | KPNA2 | ✔ | ✔ | ✔ |
| ENSG00000183763 | TRAIP | ✔ | ✔ | ✔ |
| ENSG00000183765 | CHEK2 | ✔ | ✔ | ✔ |
| ENSG00000185480 | PARPBP | ✔ | ✔ | ✔ |
| ENSG00000186280 | KDM4D | ✔ | ✔ | |
| ENSG00000196584 | XRCC2 | ✔ | ✔ | ✔ |
| ENSG00000197275 | RAD54B | ✔ | ✔ | ✔ |
| ENSG00000197299 | BLM | ✔ | ✔ | ✔ |
| ENSG00000221829 | FANCG | ✔ | ✔ | ✔ |
| ENSG00000227345 | PARG | ✔ | ✔ | |
| ENSG00000020922 | MRE11 | ✔ | | |
| ENSG00000127922 | SEM1 | ✔ | | |
| ENSG00000136504 | KAT7 | ✔ | | ✔ |
| ENSG00000140451 | PIF1 | ✔ | | |
| ENSG00000168148 | H3-4 | ✔ | | |
| ENSG00000181218 | H2AW | ✔ | | |
| ENSG00000247746 | USP51 | ✔ | | |
| ENSG00000258366 | RTEL1 | ✔ | | |
| ENSG00000270882 | H4C14 | ✔ | | |

### Discussion

Given the complexity of the repair mechanisms that respond to the distinct types of DNA lesions, it has remained elusive whether a mechanism exists that regulates the overall repair capacities of an organism. We uncovered that the *C. elegans* DREAM complex actively represses DNA repair gene expression in somatic tissues, thus curbing their repair capacity and consequently limiting developmental growth, organismal health and lifespan upon DNA damage. Our data indicate that pharmacological targeting of DREAM, e.g., of the assembly of DREAM using DYRK1A inhibitors can be applied for augmenting DNA damage resistance in human cells.

Our analysis revealed that DNA repair genes across all major DNA repair systems contain motifs in their promoters that are recognized by DREAM, resulting in their DREAM-mediated repression in the soma. Abrogating DREAM leads to de-repression of DNA repair gene expression, boosting DNA repair activity -as we demonstrated for the removal of UV-induced CPDs- and conferred significant resistance to every genotoxic agent that we tested. The recognition of DNA lesions is mediated by recognition factors that are constitutively present, such as CSB and XPC that indirectly or directly recognize helix-distorting lesions, glycosylases that recognize distinct oxidative base modifications, and DSB binding proteins such as the Ku70/80 heterodimer and the MRN complex initiating NHEJ and HRR, respectively. Transcriptional regulation of individual DNA repair genes has thus far been found as part of the DDR to further elevate levels of a specific DNA repair mechanism when responding to a specific genotoxic insult (Christmann and Kaina, 2013). In contrast, we found that abrogation of the DREAM complex leads to a constitutive induction of genes operating in all major DNA repair pathways.

DREAM is a highly conserved transcription repressor complex that regulates the induction and maintenance of cellular quiescence by repressing cell-cycle genes across multiple species (Sadasivam and DeCaprio, 2013; Uxa *et al.,* 2019). Similar to the somatic cells in *C. elegans,* quiescent mammalian cells have limited DNA repair capacities (lyama and Wilson III, 2013). For example, while some mammalian stem cell compartments such as intestinal stem cells utilize HRR, quiescent hematopoietic stem cells (HCSs) and hair follicle stem cells (HFSCs) employ NHEJ (Al zouabi and Bardin, 2020), an error-prone DSB repair mechanism, which was shown to be accountable for the increased mutagenesis during HSC aging (Mohrin *et al.,* 2010). In contrast, cell cycle entry promotes DNA repair in HSCs (Beerman *et al.,* 2014) as well as in postmitotic neurons (Schwartz *et al.,* 2007). Similarly to the DREAM targets in *C. elegans,* we found a significant number of DNA repair genes bound by DREAM in human ChIP-seq data suggesting that the function of DREAM across multiple species includes the transcriptional repression of DNA repair genes. In human cells, the DREAM complex represses cell cycle genes that are induced in the G1/S-transition and include DNA repair genes (Fischer *et al.,* 2016). Here, we established the applicability of pharmacological targeting of the DREAM complex to augment DNA damage resistance in quiescent human cells. Indeed, two independent and highly specific DYRK1A inhibitors, harmine and INDY, increased the expression of DREAM targets, including DNA repair genes, and resulted in a strikingly elevated resistance to two distinct DNA damage types (Figure 5). It is thus likely that DREAM complex inhibition in mammals could prevent DNA-damage driven conditions, such as stem cell exhaustion (McNeely *et al.,* 2019), neurodegeneration (Madabhushi, Pan and Tsai, 2014) and premature aging (Niedernhofer et *al.,* 2018). Intriguingly, the DYRK1A kinase is overexpressed in Down-Syndrome and involved in neurodegeneration in these patients, as well as in Alzheimer's, Parkinson's and Pick's disease (Ferrer *et al.,* 2005; Dowjat *et al.,* 2007; Liu *et al.,* 2008). Considering that the conserved DREAM complex is highly active in post-mitotic cells and that neurons are particularly susceptible to DNA repair defects such as TC-NER defects in Cockayne syndrome, DSB repair deficiencies in Ataxia telangiectasia, or impaired SSB repair in cerebellar ataxia (Madabhushi, Pan and Tsai, 2014), targeting of the DREAM complex provides new therapeutic avenues for a range of congenital DNA repair deficiencies in humans.

Of particular relevance to such monogenetic DNA repair deficiency syndromes, the overall elevated DNA repair gene expression revealed an interesting compensatory role between the distinct repair pathways. Abrogation of the DREAM complex enhances the removal of UV-induced DNA lesions and even suppresses defects in GG-NER and TC-NER but requires the presence of the NER machinery. These data suggest that in DREAM mutants each NER sub-pathway is elevated and their respective compensation is enhanced. Strikingly, DREAM mutants even suppress HRR deficiency, likely through elevated NHEJ. The suppression of *polh-1* mutants' sensitivity to ICLs and alkylating damage suggest that the consequence of DREAM abrogation might not necessarily be more error-prone repair. Instead, we show that DREAM mutants lead to restoration of developmental growth upon DNA damage in these mutants. Likewise, DNA damage-driven aging -in C. *elegans* induced upon exogenous DNA damage- is also alleviated in the absence of DREAM.

Our data establish the DREAM complex as a master regulator of DNA repair gene expression in somatic tissues and quiescent cells. Abrogation of the DREAM-mediated DNA repair gene repression elevates somatic repair capacities and enhances DNA damage resistance. We show that the DREAM complex restricts somatic DNA repair and its alleviation confers germline-like DNA repair capacities to the soma. Given the central role of nuclear genome stability in the aging process (Schumacher *et al.,* 2021), the DREAM complex provides a valuable target intervening at a root cause of age-related diseases. Moreover, the suppression of various DNA repair defects such as GG- and TC-NER and HRR shows that the DREAM complex provides therapeutic opportunity also in congenital DNA repair deficiency syndromes that cause developmental growth failure and premature aging.

### References

Arnoult, N. et al. (2017) 'Regulation of DNA repair pathway choice in S and G2 phases by the NHEJ inhibitor CYREN', Nature. Nature Publishing Group, 549(7673), pp. 548-552. doi: 10.1038/nature24023.
Ashburner, M. et al. (2000) 'Gene Ontology: tool for the unification of biology', Nature Genetics, 25(1), pp. 25-29. doi: 10.1038/75556.
Beerman, I. et al. (2014) 'Quiescent hematopoietic stem cells accumulate DNA damage during aging that is repaired upon entry into cell cycle', Cell Stem Cell. Elsevier Inc., 15(1), pp. 37-50. doi: 10.1016/j .stem .2014.04.016.
Bianco, J. N. and Schumacher, B. (2018) 'MPK-1/ERK pathway regulates DNA damage response during development through DAF-16/FOXO', Nucleic Acids Research, 46(12), pp. 6129-6139. doi: 10.1093/nar/gky404.
Branzei, D. and Foiani, M. (2008) 'Regulation of DNA repair throughout the cell cycle', Nature Reviews Molecular Cell Biology, 9(4), pp. 297-308. doi: 10.1038/nrm2351.
Brenner, S. (1974) 'The genetics of Caenorhabditis elegans', Genetics, 77(1), pp. 71-94.
Carbon, S. et a/. (2019) 'The Gene Ontology Resource: 20 years and still GOing strong', Nucleic Acids Research. Oxford University Press, 47(D1), pp. D330-D338. doi: 10.1093/nar/gky1055.
Ceol, C. J. et al. (2006) 'Identification and classification of genes that act antagonistically to let-60 ras signaling in Caenorhabditis elegans vulval development', Genetics, 173(2), pp. 709-726. doi: 10.1534/genetics.106.056465.
Chatterjee and Walker (2017)' Mechanisms of DNA damage, repair, and mutagenesis'. Environ Mol Mutagen 58(5), 235-263
Chen, B.-R. et al. (2021) LIN37-DREAM Prevents DNA End Resection and Homologous Recombination at DNA Double Strand Breaks in Quiescent Cells. bioRxiv. https://doi.org/10.1101/2021.04.21.440786 Christmann, M. and Kaina, B. (2013) 'Transcriptional regulation of human DNA repair genes following genotoxic stress: Trigger mechanisms, inducible responses and genotoxic adaptation', Nucleic Acids Research, 41(18), pp. 8403-8420. doi: 10.1093/nar/gkt635.
Clejan, I., Boerckel, J. and Ahmed, S. (2006) 'Developmental modulation of nonhomologous end joining in Caenorhabditis elegans', Genetics, 173(3), pp. 1301-1317. doi: 10.1534/genetics.106.058628.
Cui, M. et al. (2006) 'SynMuv Genes Redundantly Inhibit lin-3/EGF Expression to Prevent Inappropriate Vulval Induction in C. elegans', Developmental Cell, 10(5), pp. 667-672. doi: 10.1016/j .devcel.2006.04.001.
Deans, A. J. and West, S. C. (2011) 'DNA interstrand crosslink repair and cancer.', Not Rev Cancer, 11(7), pp. 467-480.
Dowjat, W. K. et al. (2007) 'Trisomy-driven overexpression of DYRK1A kinase in the brain of subjects with Down syndrome', Neuroscience Letters, 413(1), pp. 77-81. doi: 10.1016/j.neulet.2006.11.026. Duerr, J. S. (2013) 'Antibody staining in C. elegans using "freeze-cracking''', Journal of Visualized Experiments, (80), pp. 1-9. doi: 10.3791/50664.
Engeland, K. (2018), 'Cell cycle arrest through indirect transcriptional repression by p53: I have a DREAM', Cell Death & Differentiation, 25, pp. 114-132.
Fausto N. (2004) 'Liver regeneration and repair: Hepatocytes, progenitor cells, and stem cells.' Hepatology 39(6), 1477-1487.
Ferrer, I. et al. (2005) 'Constitutive DyrklA is abnormally expressed in Alzheimer disease, Down syndrome, Pick disease, and related transgenic models', Neurobiology of Disease, 20(2), pp. 392-400. doi: 10.1016/j.nbd.2005.03.020.
Fischer, M. et al. (2016) 'Integration of TP53, DREAM, MMB-FOXM1 and RB-E2F target gene analyses identifies cell cycle gene regulatory networks', Nucleic Acids Research, 44(13), pp. 6070-6086. doi: 10.1093/nar/gkw523.
Gartner, A. et al. (2000) 'A conserved checkpoint pathway mediates DNA damage--induced apoptosis and cell cycle arrest in C. elegans.', Molecular Cell, 5(3), pp. 435-443.
Göckler, N. et al. (2009) 'Harmine specifically inhibits protein kinase DYRK1A and interferes with neurite formation', FEBS Journal, 276(21), pp. 6324-6337. doi: 10.1111/j.1742-4658.2009.07346.x. Goetsch, P. D., Garrigues, J. M. and Strome, S. (2017) 'Loss of the Caenorhabditis elegans pocket protein LIN-35 reveals MuvB's innate function as the repressor of DREAM target genes', PLoS Genetics, 13(11), pp. 1-25. doi: 10.1371/journal.pgen.1007088.
Goetsch, P. D. and Strome, S. (2019) 'DREAM Interrupted : Severing MuvB from DREAM's pocket protein impairs gene repression but not DREAM assembly on chromatin', BioRxiv*.*
Gonzalo and Kreienkamp (2015) 'DNA repair defects and genome instability in Hutchinson-Gilford Progeria Syndrome.' Curr Opin Cell Biol. 34, 75-83.
Gorbunova, V. et a/. (2007) 'Changes in DNA repair during aging', Nucleic Acids Research, 35(22), pp. 7466-7474. doi: 10.1093/nar/gkm756.
Guard et al. (2019) 'The nuclear interactome of DYRK1A reveals a functional role in DNA damage repair.' Scientific Reports 9:6539
Harris, T. W. et al. (2020) 'WormBase: a modern Model Organism Information Resource', Nucleic acids research. Oxford University Press, 48(D1), pp. D762-D767. doi: 10.1093/nar/gkz920.
Hecker et al. (2017) ' Transcription factor decoy technology: A therapeutic update.' Biochemical Pharmacology, 144, 29-34.
Heinz, S. et al. (2010) 'Simple Combinations of Lineage-Determining Transcription Factors Prime cis-Regulatory Elements Required for Macrophage and B Cell Identities', Molecular Cell. Elsevier Inc., 38(4), pp. 576-589. doi: 10.1016/j.molcel.2010.05.004.
Hou, J.-W. (2009) 'Natural Course of Neonatal Progeroid Syndrome.' Pediatr. Neonatol. 50(3): 102-109. https://en.wikipedia.org/wiki/Cell_cycle
Hustedt, N. and Durocher, D. (2016) 'The control of DNA repair by the cell cycle.', Nature cell biology, 19(1), pp. 1-9.
lonescu et a/. (2012) 'DYRK1A Kinase Inhibitors with Emphasis on Cancer.' Mini-Reviews in Medicinal Chemistry 12, pp. 1315-1329.
Iwamoto et a/. (2010) 'A general chemical method to regulate protein stability in the mammalian centralnervous system.' Chem Biol. 17(9), 981-988.
lyama, T. and Wilson III, D. M. (2013) 'DNA repair mechanisms in dividing and non-dividing cells', DNA Repair, 12(8), pp. 620-636.
Jackson, S. P. and Bartek, J. (2009) 'The DNA-damage response in human biology and disease', Nature, 461(7267), pp. 1071-1078. doi: 10.1038/nature08467.
Janisiw, E. et al. (2018) 'BRCA1-BARD1 associate with the synaptonemal complex and pro-crossover factors and influence RAD-51 dynamics during Caenorhabditis elegans meiosis', PLoS Genetics, 14(11), pp. 1-30. doi: 10.1371/journal.pgen.1007653.
Johnson, N. M., Lemmens, B. B. L. G. and Tijsterman, M. (2013) 'A Role for the Malignant Brain Tumour (MBT) Domain Protein LIN-61 in DNA Double-Strand Break Repair by Homologous Recombination', PLoS Genetics. Edited by N. Maizels, 9(3), p. e1003339. doi: 10.1371/journal.pgen.1003339.
Keith, S. A. et al. (2014) 'The C. elegans healthspan and stress-resistance assay toolkit', Methods. Elsevier Inc., 68(3), pp. 476-486. doi: 10.1016/j.ymeth.2014.04.003.
Kim, D. et al. (2013) 'TopHat2: accurate alignment of transcriptomes in the presence of insertions, deletions and gene fusions', Genome Biology, 14(4), p. R36. doi: 10.1186/gb-2013-14-4-r36. Kirienko, N. V. and Fay, D. S. (2007) 'Transcriptome profiling of the C. elegans Rb ortholog reveals diverse developmental roles', Developmental Biology, 305(2), pp. 674-684. doi: 10.1016/j.ydbio.2007.02.021.
Lans, H. et al. (2010) 'Involvement of Global Genome Repair, Transcription Coupled Repair, and Chromatin Remodeling in UV DNA Damage Response Changes during Development', PLoS Genetics. Edited by N. Maizels, 6(5), p. e1000941. doi: 10.1371/journal.pgen.1000941.
Lans, H. et al. (2013) 'DNA damage leads to progressive replicative decline but extends the life span of long-lived mutant animals', Cell Death & Differentiation, 20(12), pp. 1709-1718. doi: 10.1038/cdd.2013 .126.
Latorre, I. et al. (2015) 'The DREAM complex promotes gene body H2A.Z for target repression', Genes & Development, 29(5), pp. 495-500. doi: 10.1101/gad.255810.114.
Lehner, B. et al. (2006) 'Loss of LIN-35, the Caenorhabditis elegans ortholog of the tumor suppressor p105Rb, results in enhanced RNA interference.', Genome biology, 7(1), p. R4. doi: 10.1186/gb-2006-7-1-r4.
Lévy et al. (1999) 'Analysis of a conditional degradation signal in yeast and mammalian cells.' Eur J Biochem. 259(1-2), 244-252.
Liang and Zhang (2013) 'Genetic and epigenetic variations in iPSCs: potential causes and implications for application.' Cell Stem Cell 13(2), 149-159.
Litovchick, L. et al. (2007) 'Evolutionarily Conserved Multisubunit RBL2/p130 and E2F4 Protein Complex Represses Human Cell Cycle-Dependent Genes in Quiescence', Molecular Cell, 26(4), pp. 539-551. doi: 10.1016/j.molcel.2007.04.015.
Litovchick, L. et al. (2011) 'DYRK1A protein kinase promotes quiescence and senescence through DREAM complex assembly', Genes Dev, 25(8), pp. 801-813.
Liu, F. et al. (2008) 'Overexpression of DyrklA contributes to neurofibrillary degeneration in Down syndrome', The FASEB Journal, 22(9), pp. 3224-3233. doi: 10.1096/fj.07-104539.
Love, M. I., Huber, W. and Anders, S. (2014) 'Moderated estimation of fold change and dispersion for RNA-seq data with DESeq2', Genome Biology, 15(12), pp. 1-21. doi: 10.1186/s13059-014-0550-8. Lu, X. and Horvitz, H. R. (1998) 'Lin-35 and lin-53, two genes that antagonize a C. elegans Ras pathway, encode proteins similar to Rb and its binding protein RbAp48', Cell, 95(7), pp. 981-991. doi: 10.1016/S0092 -8674(00)81722-5.
Lynch, M. (2010) 'Evolution of the mutation rate', Trends Genet, 26(8), pp. 345-352. doi: 10.1016/j.tig.2010.05.003.Evolution.
Madabhushi, R., Pan, L. and Tsai, L.-H. (2014) 'DNA Damage and Its Links to Neurodegeneration', Neuron, 83(2), pp. 266-282. doi: 10.1016/j.neuron.2014.06.034.
Mannefeld, M., Klassen, E. and Gaubatz, S. (2009) 'B-MYB is required for recovery from the DNA damage-induced G2 checkpoint in p53 mutant cells', Cancer Research, 69(9), pp. 4073-4080. doi: 10.1158/0008-5472. CAN-08-4156.
Maynard, S. et al. (2008) 'Human Embryonic Stem Cells Have Enhanced Repair of Multiple Forms of DNA Damage', Stem Cells, 26(9), pp. 2266-2274. doi: 10.1634/stemcells.2007-1041.
McNeely, T. et a/. (2019) 'DNA damage in aging, the stem cell perspective', Human Genetics. Springer Berlin Heidelberg, (0123456789). doi: 10.1007/s00439-019-02047-z.
Milholland, B. et al. (2017) 'Differences between germline and somatic mutation rates in humans and mice', Nature Communications. Nature Publishing Group, 8(1), p. 15183. doi: 10.1038/ncomms15183.
Mohrin, M. et al. (2010) 'Hematopoietic stem cell quiescence promotes error-prone DNA repair and mutagenesis', Cell Stem Cell, 7(2), pp. 174-185. doi: 10.1016/j.stem.2010.06.014.
Momcilovic, O. et al. (2010) 'DNA damage responses in human induced pluripotent stem cells and embryonic stem cells', PLoS ONE, 5(10). doi: 10.1371/journal.pone.0013410.
Mueller, M. M. et al. (2014) 'DAF-16/FOXO and EGL-27/GATA promote developmental growth in response to persistent somatic DNA damage', Nature Cell Biology, 16(12), pp. 1168-1179. doi: 10.1038/ncb3071.
Müller, G. A. et al. (2012) 'The CHR promoter element controls cell cycle-dependent gene transcription and binds the DREAM and MMB complexes', Nucleic Acids Research, 40(4), pp. 1561-1578. doi: 10.1093/nar/gkr793.
Müller, G. A. and Engeland, K. (2010) 'The central role of CDE/CHR promoter elements in the regulation of cell cycle-dependent gene transcription', FEBS Journal, 277(4), pp. 877-893. doi: 10. 1111/j.1742-4658.2009.07508 .x.
Niedernhofer, L. J. et a/. (2018) 'Nuclear Genomic Instability and Aging',Annu Rev Biochem, 87(1), pp. 295-322.
Nussbaum-Krammer, C. I. et al. (2015) 'Investigating the Spreading and Toxicity of Prion-like Proteins Using the Metazoan Model Organism <em>C. elegans</em>', Journal of Visualized Experiments, (95), p. 52321. doi: 10.3791/52321.
Ogawa, Y. et al. (2010) 'Development of a novel selective inhibitor of the Down syndrome-related kinase DyrklA', Nature Communications. Nature Publishing Group, 1(7). doi: 10.1038/ncomms1090. Osterloh, L. et al. (2007) 'The human synMuv-like protein LIN-9 is required for transcription of G2/M genes and for entry into mitosis', EMBO Journal, 26(1), pp. 144-157. doi: 10.1038/sj.emboj.7601478. Ou, H.-L. et a/. (2019) 'Somatic Niche Cells Regulate the CEP-1/p53-Mediated DNA Damage Response in Primordial Germ Cells', Developmental Cell, 50(2), pp. 167-183.e8. doi: 10.1016/j .devcel.2019.06 .012.
Patro, R. et a/. (2017) 'Salmon provides fast and bias-aware quantification of transcript expression', Nature Methods. Nature Publishing Group, 14(4), pp. 417-419. doi: 10.1038/nmeth.4197.
Reinke, V. et al. (2004) 'Genome-wide germline-enriched and sex-biased expression profiles in Caenorhabditis elegans', Development, 131(2), pp. 311-323. doi: 10.1242/dev.00914.
Rieckher, M. et al. (2017) 'A simple answer to complex questions: Caenorhabditis elegansas an experimental model for examining the DNA damage response and disease genes', Journal of Cellular Physiology, 233(4), pp. 2781-2790. doi: 10.1002/jcp.25979.
Robinson, M. D., McCarthy, D. J. and Smyth, G. K. (2009) 'edgeR: A Bioconductor package for differential expression analysis of digital gene expression data', Bioinformatics, 26(1), pp. 139-140. doi: 10.1093/bioinformatics/btp616.
Roerink, S. F. et al. (2012) 'A Broad Requirement for TLS Polymerases η and K, and Interacting Sumoylation and Nuclear Pore Proteins, in Lesion Bypass during C. elegans Embryogenesis', PLoS Genetics. Edited by S. Jinks-Robertson, 8(6), p. e1002800. doi: 10.1371/journal.pgen.1002800. Sadasivam, S. and DeCaprio, J. A. (2013) 'The DREAM complex: master coordinator of cell cycle-dependent gene expression', Nature Reviews Cancer, 13(8), pp. 585-595. doi: 10.1038/nrc3556. Sadasivam, S., Duan, S. and DeCaprio, J. a. (2012) 'The MuvB complex sequentially recruits B-Myb and FoxMl to promote mitotic gene expression', Genes and Development, 26(5), pp. 474-489. doi: 10.1101/gad.181933 .111.
Saretzki, G. et al. (2004) 'Stress Defense in Murine Embryonic Stem Cells Is Superior to That of Various Differentiated Murine Cells', Stem Cells, 22(6), pp. 962-971. doi: 10.1634/stemcells.22-6-962. Schindelin, J. et al. (2012) 'Fiji: An open-source platform for biological-image analysis', Nature Methods, 9(7), pp. 676-682. doi: 10.1038/nmeth.2019.
Schmittgen, T. D. and Livak, K. J. (2008) 'Analyzing real-time PCR data by the comparative CT method', Nature Protocols, 3(6), pp. 1101-1108. doi: 10.1038/nprot.2008.73.
Schumacher, B. et al. (2021) 'The central role of DNA damage in the ageing process', Nature. Springer US, 592(April), pp. 695-703. doi: 10.1038/s41586-021-03307-7.
Schwartz, E. I. et al. (2007) 'Cell cycle activation in postmitotic neurons is essential for DNA repair', Cell Cycle, 6(3), pp. 318-329. doi: 10.4161/cc.6.3.3752.
Seabold, S. and Perktold, J. (2010) 'Statsmodels: Econometric and Statistical Modeling with Python', in Proceedings of the 9th Python in Science Conference, pp. 92-96. doi: 10.25080/Majora-92bf1922-011.
Sierra, H. et al. (2015) 'Confocal Imaging-Guided Laser Ablation of Basal Cell Carcinomas: An Ex Vivo Study', Journal of Investigative Dermatology, 135(2), pp. 612-615. doi: 10.1038/jid.2014.371.
da Silva, P. F. L. and Schumacher, B. (2019) 'DNA damage responses in ageing', Open Biology, 9(11), p. 190168. doi: 10.1098/rsob.190168.
Soll, J. M., Sobol, R. W. and Mosammaparast, N. (2017) 'Regulation of DNA Alkylation Damage Repair: Lessons and Therapeutic Opportunities', Trends in Biochemical Sciences. Elsevier Ltd, 42(3), pp. 206-218. doi: 10.1016/j.tibs.2016.10.001.
Soneson, C., Love, M. I. and Robinson, M. D. (2016) 'Differential analyses for RNA-seq: transcript-level estimates improve gene-level inferences', F1000Research, 4, p. 1521. doi: 10.12688/f1000research.7563.2.
Stankunas et al. (2003) 'Conditional protein alleles using knockin mice and a chemical inducer of dimerization.' Mol Cell. 12(6), 1615-1624.
Tabuchi, T. M. et al. (2011) 'Chromosome-biased binding and gene regulation by the caenorhabditis elegans DRM complex', PLoS Genetics, 7(5). doi: 10.1371/journal.pgen.1002074.
Tapia and Schöler (2016) ' Molecular Obstacles to Clinical Translation of iPSCs.' Cell Stem Cell 19, 298-309.
Tichy, E. D. and Stambrook, P. J. (2008) 'DNA repair in murine embryonic stem cells and differentiated cells', Experimental Cell Research, 314(9), pp. 1929-1936. doi: 10.1016/j.yexcr.2008.02.007. Trapnell, C. et al. (2010) 'Transcript assembly and quantification by RNA-Seq reveals unannotated transcripts and isoform switching during cell differentiation', Nature Biotechnology. Nature Publishing Group, 28(5), pp. 511-515. doi: 10.1038/nbt.1621.
Unhavaithaya, Y. et al. (2002) 'MEP-1 and a Homolog of the NURD Complex Component Mi-2 Act Together to Maintain Germline-Soma Distinctions in C. elegans', Cell, 111(7), pp. 991-1002. doi: 10.1016/S0092-8674(02)01202-3.
Uxa, S. et al. (2019) 'DREAM and RB cooperate to induce gene repression and cell-cycle arrest in response to p53 activation', Nucleic acids research, 47(17), pp. 9087-9103. doi: 10.1093/nar/gkz635. Vermezovic, J. et al. (2012) 'Differential regulation of DNA damage response activation between somatic and germline cells in Caenorhabditis elegans', Cell Death & Differentiation, 19(11), pp. 1847-1855. doi: 10.1038/cdd.2012.69.
Vitale et al. (2017) ' DNA Damage in Stem Cells.' Molecular Cell 66, 306-319
Virtanen, P. et al. (2020) 'SciPy 1.0: fundamental algorithms for scientific computing in Python', Nature Methods, 17(3), pp. 261-272. doi: 10.1038/s41592-019-0686-2.
Wagle, P., Nikolić, M. and Frommolt, P. (2015) 'QuickNGS elevates Next-Generation Sequencing data analysis to a new level of automation', BMC Genomics, 16(1), pp. 1-8. doi: 10.1186/sl2864-015-1695-x.
Wang, D. et al. (2005) 'Somatic misexpression of germline P granules and enhanced RNA interference in retinoblastoma pathway mutants', Nature, 436(7050), pp. 593-597. doi: 10.1038/nature04010. Wilson, D. M. et al. (2017) 'Systematic analysis of DNA crosslink repair pathways during development and aging in Caenorhabditis elegans', Nucleic Acids Research, 45(16), pp. 9467-9480. doi: 10.1093/nar/gkx660.
WO 2020/069418 A1
Yates, A. D. et al. (2020) 'Ensembl 2020', Nucleic acids research, 48(D1), pp. D682-D688. doi: 10.1093/nar/gkz966.
Yoon et al. (2020) Small Molecule Inhibitors of DYRK1A Identified by Computational and Experimental Approaches Int. J. Mol. Sci.
Al zouabi, L. and Bardin, A. J. (2020) 'Stem Cell DNA Damage and Genome Mutation in the Context of Aging and Cancer Initiation', Cold Spring Harbor Perspectives in Biology, p. a036210. doi: 10.1101/csh perspect.a036210

## Claims

1. An inhibitor of DREAM complex assembly and/or function for use in repairing DNA damage in a cell of a subject.

2. The inhibitor of DREAM complex assembly and/or function for use of claim 1,
wherein the inhibitor of DREAM complex assembly and/or function is selected from the group comprising an inhibitory nucleic acid, an antibody and a small molecule inhibitor.

3. The inhibitor of DREAM complex assembly and/or function for use of any of claims 1 or 2, wherein the inhibitor of DREAM complex assembly is an inhibitory nucleic acid inhibiting the expression of a component of the DREAM complex or an antibody to a component of the DREAM complex.

4. An inhibitor of DREAM complex assembly and/or function for use in treating and/or preventing a condition associated with DNA damage in a cell in a subject, wherein the condition is selected from the group comprising age-related diseases and symptoms of ageing, a progeroid syndrome, acute radiation syndrome, chronic radiation syndrome, Xeroderma pigmentosum, Nijmegen breakage syndrome, Fanconi anemia and ataxia, and wherein DNA damage in a cell of a subject is repaired, preferably, for use of claim 1.

5. A method for obtaining at least one cell with reduced DNA damage, wherein the method comprises steps of:
a) providing at least one cell, optionally, from a sample from a subject, and
b) treating said at least one cell with an inhibitor of DREAM complex assembly and/or function to repair DNA damage in said cell,
wherein, preferably, the method is an *in vitro* or *ex vivo* method.

6. The inhibitor of DREAM complex assembly and/or function for use of any of claims 1-4 or the method of claim 5, wherein the inhibitor of DREAM complex assembly and/or function is selected from the group comprising an inhibitory nucleic acid, an antibody and a small molecule inhibitor.

7. The inhibitor of DREAM complex assembly and/or function for use of any of claims 1-4 or 6 or the method of any of claims 5 or 6, wherein the inhibitor of DREAM complex assembly is an inhibitory nucleic acid inhibiting the expression of a component of the DREAM complex, wherein, preferably, the inhibitory nucleic acid is an inhibitory RNA.

8. The inhibitor of DREAM complex assembly and/or function for use of any of claims 1-4 or 6 or the method of any of claims 5 or 6, wherein the inhibitor of DREAM complex assembly is an antibody to a component of the DREAM complex.

9. The inhibitor of DREAM complex assembly and/or function for use of any of claims 1-4 or 6 or the method of any of claims 5 or 6, wherein the inhibitor of DREAM complex assembly is a DYRK1A inhibitor selected from the group consisting of an antibody, an inhibitory RNA, and a small molecule inhibitor selected from the group comprising epigallocatechin-3-gallate (EGCG), harmine, (1Z)-1-(3-ethyl-5-hydroxy-2(3H)-benzothiazoylidene)-2-propanone (INDY), 3,5-di(polyhydroxyaryl)-7-azaindole (DANDY), (3-(4-fluorophenyl)-5-(3,4-dihydroxyphenyl)-1H-pyrrolo[2,3-b]pyridine (F-DANDY), folding intermediate-selective inhibitor of DYRK1A (FINDY), GNF4877, Roscovitine, Purvalanol A, 4,5,6,7-tetrabromo-1*H*-benzotriazole (TBB), CR8, quinazolinone, quinalizarin, Apigenin, Flavokavain A, Emodin, a meriolin, Ageladine A and Leucettine L41.

10. The inhibitor of DREAM complex assembly and/or function for use of any of claims 1-4 or 6-9 or the method of any of claims 5-9, wherein the DNA damage is selected from the group comprising cyclobutane pyrimidine dimers (CPDs), pyrimidine (6-4) pyrimidone photoproducts (6-4PPs), DNA alkylations, base oxidations, base deami-nations, interstrand crosslinks (ICLs), DNA mismatches, base loss, DNA single-strand breaks (SSBs) and DNA double-strand breaks (DSBs).

11. The inhibitor of DREAM complex assembly and/or function for use of any of claims 1-4 or 6-10 or the method of any of claims 5-10, wherein the cell is a quiescent cell selected from the group comprising a tissue-specific stem cell, a mature hepatocyte, and a fully differentiated cell selected from the group comprising a neuronal cell and a kidney cell.

12. The inhibitor of DREAM complex assembly and/or function for use of any of claims 1-4 or 6-11 or the method of any of claims 5-11, wherein the inhibitor of DREAM complex assembly is targeted to the cell in the subject using a cell-specific vector.

13. The inhibitor of DREAM complex assembly and/or function for use of any of claims 4 or 6-12, wherein the progeroid syndrome is selected from the group comprising Werner syndrome, Bloom syndrome, Rothmund-Thomson syndrome, Cockayne syndrome, trichothiodystrophy, combined xeroderma pigmentosum-Cockayne syndrome, Wiedemann-Rautenstrauch syndrome or Hutchinson-Gilford progeria syndrome.

14. The inhibitor of DREAM complex assembly and/or function for use of any of claims 4 or 6-13, wherein the age-related disease is cancer, neurodegeneration and stem cell exhaustion.

15. At least one cell obtainable by the method of any of claims 5-12,
wherein, optionally, the cell is a stem cell, preferably a pluripotent stem cell, such as an induced pluripotent stem cell.

16. A pharmaceutical composition comprising the at least one cell of claim 15 and, optionally, at least one pharmaceutically acceptable excipient.
